# EUROPEAN PATENT APPLICATION

(11) **EP 3 992 295 A1**
(43) Date of publication of application: **04.05.2022**
(21) Application number: 20841258.5
(22) Date of filing: 23.06.2020
(51) Int. Cl.: C12N 15/62, C07K 14/415, C07K 19/00, C12N 1/15, C12N 1/19, C12N 1/21, C12N 5/10, C12N 9/10, C12N 15/29, C12N 15/54, C12N 15/63, C12P 5/00

(54) **FUSION PROTEIN, SUBSTANCE PRODUCTION METHOD, VECTOR, TRANSFORMED CELL, PNEUMATIC TIRE PRODUCTION METHOD, AND RUBBER PRODUCT PRODUCTION METHOD**

(30) Priority: 16.07.2019 JP 2019131427
(71) Applicant: Sumitomo Rubber Industries, Ltd., Kobe-shi, Hyogo 651-0072 (JP); Tohoku University, Aoba-ku Sendai-shi Miyagi 980-8577 (JP); Kanazawa University, Kanazawa-shi Ishikawa 920-1192 (JP); National University Corporation Saitama University, Saitama City, Saitama 338-8570 (JP)
(72) Inventor: YAMAGUCHI, Haruhiko, Kobe-shi, Hyogo 651-0072 (JP); INOUE, Yukino, Kobe-shi, Hyogo 651-0072 (JP); TAKAHASHI, Seiji, Sendai-shi, Miyagi 980-8577 (JP); NAKAYAMA, Toru, Sendai-shi, Miyagi 980-8577 (JP); YAMASHITA, Satoshi, Kanazawa-shi, Ishikawa 920-1192 (JP); TOZAWA, Yuzuru, Saitama-shi, Saitama 338-8570 (JP)
(74) Representative: Manitz Finsterwald Patent- und Rechtsanwaltspartnerschaft mbB
(86) International application number: PCT/JP2020/024525
(87) International publication number: WO 2021/010101

(57) **Abstract**

Objects are to provide: a fusion protein capable of binding to lipid droplets while having an enzymatic activity to synthesize a hydrophobic compound; a method for producing a substance including accumulating a hydrophobic compound in lipid droplets using the fusion protein; a vector which can enhance production of a hydrophobic compound when it is introduced into cells using genetic recombination techniques; and a transgenic cell into which the vector or a gene coding for the fusion protein has been introduced. The disclosure relates to a fusion protein having an amino acid sequence (first amino acid sequence) capable of binding to lipid droplets, and an amino acid sequence (second amino acid sequence) having an enzymatic activity to synthesize a hydrophobic compound, the first and second amino acid sequences being fused via a linker sequence (third amino acid sequence) while maintaining the enzymatic activity of the second amino acid sequence.

## Description

### TECHNICAL FIELD

The present disclosure relates to a fusion protein, a method for producing a substance, a vector, a transgenic cell, a method for producing a pneumatic tire, and a method for producing a rubber product.

### BACKGROUND ART

Nowadays, natural rubber (a type of polyisoprenoid) for use in industrial rubber products may be obtained by cultivating rubber-producing plants, such as para rubber trees (*Hevea brasiliensis*) belonging to the family *Euphorbiaceae* or Indian rubber trees (*Ficus elastica*) belonging to the family *Moraceae,* to cause the laticifer cells of the plants to biosynthesize natural rubber, and then manually harvesting the natural rubber from the plants.

At present, *Hevea brasiliensis* is virtually the only source of natural rubber used in industrial rubber products. *Hevea brasiliensis* is a plant that can grow only in limited regions, including Southeast Asia and South America. Moreover, *Hevea brasiliensis* trees take about seven years from planting to grow mature enough to yield rubber, and they yield natural rubber only for a period of 20 to 30 years. Demand for natural rubber is expected to increase in years to come, especially in developing countries, but for the reasons discussed above it is difficult to greatly increase natural rubber production from *Hevea brasiliensis.* Therefore, there has been a concern that natural rubber resources might dry up, and it has been desirable to provide stable natural rubber sources other than mature *Hevea brasiliensis* trees and to improve productivity of natural rubber from *Hevea brasiliensis.*

Natural rubber has a cis-1,4-polyisoprene structure formed of isopentenyl diphosphate (IPP) as an elementary unit. This structure suggests that cis-prenyltransferase (CPT) may be involved in natural rubber biosynthesis. For example, several CPTs have been found in *Hevea brasiliensis,* including Hevea rubber transferase 1 (HRT1) and Hevea rubber transferase 2 (HRT2) (see, for example, Non-Patent Literatures 1 and 2). It is also known that rubber synthesis may be reduced in a dandelion species, *Taraxacum brevicorniculatum,* by suppressing the CPT expression (see, for example, Non-Patent Literature 3).

### CITATION LIST

### NON-PATENT LITERATURE

Non-Patent Literature 1: Rahaman et al., BMC Genomics, 2013, vol. 14
Non-Patent Literature 2: Asawatreratanakul et al., EuropeanJournal of Biochemistry, 2003, vol. 270, 4671-4680
Non-Patent Literature 3: Post et al., Plant Physiology, 2012, vol. 158, 1406-1417
Non-Patent Literature 4: Karine et al., Biochimica et Biophysica Acta 1838 (2014), 287-299

### SUMMARY OF DISCLOSURE

### TECHNICAL PROBLEM

As discussed above, there have been needs to develop stable natural rubber sources other than mature *Hevea brasiliensis* trees and to improve productivity of natural rubber from *Hevea brasiliensis.*

As a solution to these problems, the present inventors discovered that a polyisoprenoid (natural rubber) may be accumulated in rubber particles by binding a cis-prenyltransferase (CPT) family protein, which is a rubber synthase, to rubber particles, and causing an enzymatic reaction (see, for example, WO 2017/002818).

As a result of extensive research and experimentation, the present inventors have found that the above approach is disadvantageous in that the use of rubber particles limits the range of applications because when considering rubber synthesis in cells, limited cells (plants) can produce rubber particles.

Then, the present inventors have conducted extensive research and experimentation to arrive at the use of lipid droplets present in all plants, instead of rubber particles possessed only by special plants.

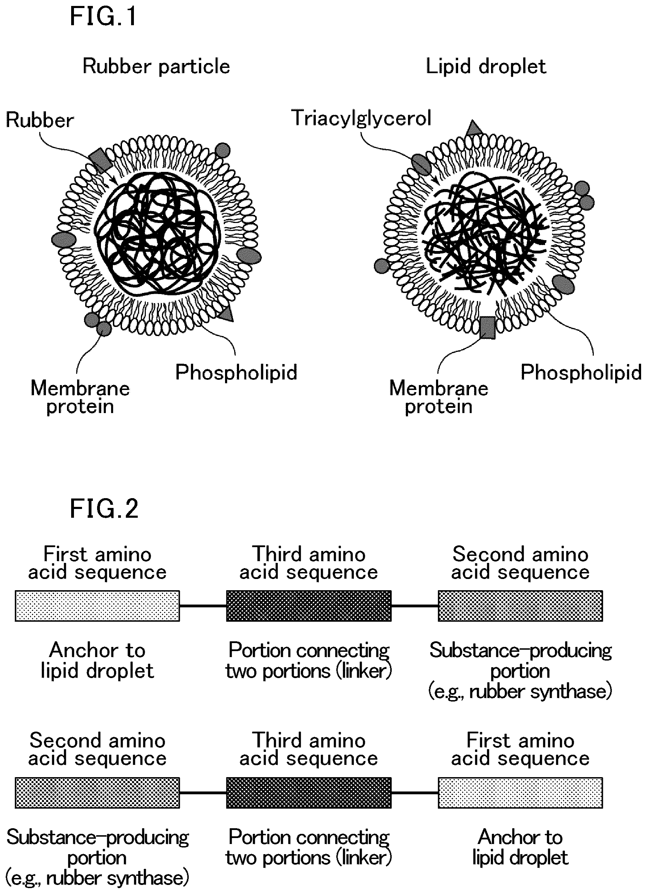

As shown in Fig. 1, a lipid droplet has a membrane structure including phospholipids and membrane proteins (for example, oleosins or lipid droplet-associated protein (LDAP)/small rubber particle protein (SRPP) family proteins) and stores triacylglycerols therein.

Rubber particles and lipid droplets are similar in that: the membranes have a single lipid membrane structure; hydrophobic substances are stored inside; and multiple proteins exist on the membranes. Thus, the inventors expected that any enzyme capable of binding to rubber particles could also bind to lipid droplets. Actually, however, it was found that enzymes capable of binding to rubber particles do not bind to lipid droplets.

The present disclosure aims to solve the new problem found by the present inventors and provide a fusion protein capable of binding to lipid droplets while having an enzymatic activity to synthesize a hydrophobic compound, a method for producing a substance including accumulating a hydrophobic compound in lipid droplets using the fusion protein, a vector which can enhance production of a hydrophobic compound when it is introduced into cells using genetic recombination techniques, and a transgenic cell into which the vector or a gene coding for the fusion protein has been introduced.

### SOLUTION TO PROBLEM

The present inventors have investigated why enzymes capable of binding to rubber particles, such as rubber synthases, do not bind to lipid droplets. As a result, they have arrived at a hypothesis that the reason is the difference in the type of lipid forming the membrane between rubber particles and lipid droplets, or in other words that enzymes such as rubber synthases hardly bind to lipid droplets due to the difference in the type of lipid forming the membrane between rubber particles and lipid droplets.

It is reported that the binding ability of rubber elongation factors (REFs) capable of binding to rubber particles depends on the type of lipid (Non-Patent Literature 4).

Thus, an attempt was made to fuse an amino acid sequence having an enzymatic activity to synthesize a hydrophobic compound with an amino acid sequence capable of binding to lipid droplets as an anchor (for example, an amino acid sequence derived from a protein originally present in lipid droplets) in order to enhance the lipid droplet-binding ability of an enzyme that has an enzymatic activity to synthesize a hydrophobic compound and that itself is not capable of binding to lipid droplets, such as a rubber synthase.

However, even when an amino acid sequence having an enzymatic activity to synthesize a hydrophobic compound was fused with an amino acid sequence capable of binding to lipid droplets, binding to lipid droplets in some cases failed depending on the type of lipid droplet-binding protein used, or the enzymatic activity on lipid droplets was insufficient depending on the fusion method.

The inventors have investigated why binding to lipid droplets in some cases failed depending on the lipid droplet-binding protein used, and then found that lipid droplet-binding proteins are classified into class I proteins which are originally bound to the membrane upon the formation of lipid droplets and class II proteins which bind to the formed lipid droplets, and that in order to bind proteins to the formed lipid droplets, it is necessary to use the class II proteins because the class I proteins cannot bind to the formed lipid droplets.

The inventors have also investigated why the enzymatic activity of some fusion proteins was deactivated, and then found that fusion proteins provided with a linker sequence between the amino acid sequences, specifically, fusion proteins having a structure as shown in Fig. 2, can exhibit sufficient enzymatic activity on lipid droplets. These findings have led to the completion of the present disclosure.

Specifically, the present disclosure relates to a fusion protein, having: an amino acid sequence (first amino acid sequence) capable of binding to lipid droplets; and an amino acid sequence (second amino acid sequence) having an enzymatic activity to synthesize a hydrophobic compound, the first amino acid sequence and the second amino acid sequence being fused via a linker sequence (third amino acid sequence) while maintaining the enzymatic activity of the second amino acid sequence.

Preferably, the first amino acid sequence is an amino acid sequence derived from a protein capable of binding to lipid droplets which is a class II protein.

Preferably, the second amino acid sequence is an amino acid sequence derived from an enzyme that has an enzymatic activity to synthesize a hydrophobic compound and that itself is not capable of binding to lipid droplets.

Preferably, the first amino acid sequence is an amino acid sequence derived from a lipid droplet-associated protein (LDAP)/small rubber particle protein (SRPP) family protein.

Preferably, the first amino acid sequence is an amino acid sequence derived from a LDAP/SRPP family protein of plant origin.

Preferably, the first amino acid sequence is an amino acid sequence derived from a LDAP/SRPP family protein derived from at least one selected from the group consisting of plants of the genera *Persea*, *Hevea*, and *Taraxacum.*

Preferably, the first amino acid sequence is an amino acid sequence derived from a LDAP/SRPP family protein derived from at least one plant selected from the group consisting of *Persea americana*, *Hevea brasiliensis*, and *Taraxacum kok-saghyz.*

Preferably, the second amino acid sequence is an amino acid sequence derived from a prenyltransferase family protein.

Preferably, the second amino acid sequence is an amino acid sequence derived from a cis-prenyltransferase family protein.

Preferably, the second amino acid sequence is an amino acid sequence derived from a cis-prenyltransferase family protein derived from a plant of the genus *Hevea* or *Taraxacum.*

Preferably, the third amino acid sequence consists of four or more amino acids.

Preferably, the third amino acid sequence consists of 20 to 30 amino acids.

Preferably, the third amino acid sequence is any one of the following amino acid sequences [1] to [3]:
[1] the amino acid sequence represented by SEQ ID NO:5;
[2] an amino acid sequence containing one or more amino acid substitutions, deletions, insertions, and/or additions relative to the amino acid sequence represented by SEQ ID NO:5; and
[3] an amino acid sequence with at least 80% sequence identity to the amino acid sequence represented by SEQ ID NO:5.

The present disclosure also relates to a method for producing a substance, the method including
binding the above fusion protein to lipid droplets, and
accumulating a product in the lipid droplets by the enzymatic activity of the second amino acid sequence.

The present disclosure also relates to a vector into which a gene coding for the above fusion protein has been introduced.

The present disclosure also relates to a transgenic cell into which a gene coding for the above fusion protein has been introduced.

The present disclosure also relates to a method for producing a substance, the method including
using the above transgenic cell to accumulate a product in lipid droplets in the cell by the enzymatic activity of the second amino acid sequence.

The present disclosure also relates to a method for producing a pneumatic tire, the method including:
producing a polyisoprenoid by the above method for producing a substance;
kneading the polyisoprenoid with an additive to obtain a kneaded mixture;
forming a green tire from the kneaded mixture; and
vulcanizing the green tire.

The present disclosure also relates to a method for producing a rubber product, the method including:
producing a polyisoprenoid by the above method for producing a substance;
kneading the polyisoprenoid with an additive to obtain a kneaded mixture;
forming a raw rubber product from the kneaded mixture; and
vulcanizing the raw rubber product.

### ADVANTAGEOUS EFFECTS OF DISCLOSURE

The fusion protein according to the present disclosure has an amino acid sequence (first amino acid sequence) capable of binding to lipid droplets, and an amino acid sequence (second amino acid sequence) having an enzymatic activity to synthesize a hydrophobic compound, the first amino acid sequence and the second amino acid sequence being fused via a linker sequence (third amino acid sequence) while maintaining the enzymatic activity of the second amino acid sequence. Thus, the fusion protein is capable of binding to lipid droplets while having an enzymatic activity to synthesize a hydrophobic compound. Even if the second amino acid sequence needs a cofactor to exhibit enzymatic activity, the fused first amino acid sequence and third amino acid sequence do not inhibit it from assisting in the activation.

Thus, when the fusion protein is used with lipid droplets, the fusion protein binds to the lipid droplets, and the hydrophobic compound synthesized by the fusion protein can be accumulated in the lipid droplets.

Moreover, the fusion protein which can be suitably used in cells containing lipid droplets can be used in a wide range of cells to enhance production of the hydrophobic compound in the wide range of cells because lipid droplets exist in both prokaryotes and eukaryotes.

Moreover, a vector into which a gene coding for the fusion protein has been introduced can enhance production of the hydrophobic compound when it is introduced into cells using genetic recombination techniques. In a transgenic cell into which the vector or a gene coding for the fusion protein has been introduced, the production of the hydrophobic compound can be enhanced.

The method for producing a pneumatic tire of the present disclosure includes producing a polyisoprenoid by the method for producing a substance of the present disclosure, kneading the polyisoprenoid with an additive to obtain a kneaded mixture, forming a green tire from the kneaded mixture, and vulcanizing the green tire. This method produces a pneumatic tire from a polyisoprenoid produced by a method that produces a polyisoprenoid with high productivity. Thus, plant resources can be effectively used, and environmentally friendly pneumatic tires can be produced.

The method for producing a rubber product of the present disclosure includes producing a polyisoprenoid by the method for producing a substance of the present disclosure, kneading the polyisoprenoid with an additive to obtain a kneaded mixture, forming a raw rubber product from the kneaded mixture, and vulcanizing the raw rubber product. This method produces a rubber product from a polyisoprenoid produced by a method that produces a polyisoprenoid with high productivity. Thus, plant resources can be effectively used, and environmentally friendly rubber products can be produced.

### BRIEF DESCRIPTION OF DRAWINGS

Fig. 1 is a schematic diagram illustrating the structures of a rubber particle and a lipid droplet.
Fig. 2 is a schematic diagram showing exemplary fusion proteins of the present disclosure.
Fig. 3 is a schematic diagram illustrating part of a polyisoprenoid biosynthesis pathway.
Fig. 4 is an outline diagram showing the results of multiple sequence alignment of CPT family proteins derived from various organisms.
Fig. 5 is a diagram showing a summary of an exemplary method for preparing lipid droplets.
Fig. 6 is a diagram showing exemplary SDS-PAGE results.
Fig. 7 is a diagram showing exemplary SDS-PAGE results.
Fig. 8 is a diagram showing exemplary SDS-PAGE results.

### DESCRIPTION OF EMBODIMENTS

### <Fusion protein>

The fusion protein of the present disclosure has an amino acid sequence (first amino acid sequence) capable of binding to lipid droplets, and an amino acid sequence (second amino acid sequence) having an enzymatic activity to synthesize a hydrophobic compound. The first amino acid sequence and the second amino acid sequence are fused via a linker sequence (third amino acid sequence) while maintaining the enzymatic activity of the second amino acid sequence.

As described earlier, the fusion protein of the present disclosure has a structure as shown in Fig. 2 and therefore is capable of binding to lipid droplets while having an enzymatic activity to synthesize a hydrophobic compound and can exhibit sufficient enzymatic activity on the lipid droplets. This allows an enzymatic reaction which in nature cannot occur on lipid droplets to occur on the lipid droplets.

Here, the fusion protein of the present disclosure may have the first amino acid sequence, the third amino acid sequence, and the second amino acid sequence in the stated order from the N-terminal side, or may have the first amino acid sequence, the third amino acid sequence, and the second amino acid sequence in the stated order from the C-terminal side, as shown in Fig. 2.

First, the first amino acid sequence, the second amino acid sequence, and the third amino acid sequence are described. Firstly, the second amino acid sequence that has an enzymatic activity is described.

### (Second amino acid sequence)

The second amino acid sequence is an amino acid sequence having an enzymatic activity to synthesize a hydrophobic compound.

Although the amino acid sequence having an enzymatic activity to synthesize a hydrophobic compound may be any amino acid sequence that has an enzymatic activity to synthesize a hydrophobic compound, it is preferably an amino acid sequence derived from an enzyme having an enzymatic activity to synthesize a hydrophobic compound, more preferably an amino acid sequence derived from an enzyme that has an enzymatic activity to synthesize a hydrophobic compound and that itself is not capable of binding to lipid droplets. Here, since it is sufficient that the amino acid sequence derived from an enzyme having an enzymatic activity to synthesize a hydrophobic compound has such enzymatic activity, it may be the entire amino acid sequence of the enzyme having an enzymatic activity to synthesize a hydrophobic compound or a part of the amino acid sequence, e.g., from which a transport signal sequence has been removed.

The hydrophobic compound may be any hydrophobic compound that can be stored in lipid droplets. Examples include isoprenoid compounds, fatty acids, fat-soluble vitamins, and hydrophobic polymers (excluding isoprenoid compounds). Isoprenoid compounds are preferred among these, with polyisoprenoids (natural rubber) being more preferred.

Non-limiting examples of enzymes corresponding to the above-mentioned hydrophobic compounds (enzymes having an enzymatic activity to synthesize the hydrophobic compounds) include rubber synthases, lycopene cyclases, and polyhydroxyalkanoate (PHA) synthases. Rubber synthases are preferred among these.

Rubber synthases themselves are usually not capable of binding to lipid droplets and thus correspond to enzymes which have an enzymatic activity to synthesize a hydrophobic compound and which themselves are not capable of binding to lipid droplets.

Preferred examples of rubber synthases are prenyltransferase family proteins. Prenyltransferase family proteins include cis-prenyltransferase family proteins and trans-prenyltransferase family proteins, with cis-prenyltransferase family proteins being preferred. In other words, the second amino acid sequence is preferably an amino acid sequence derived from a prenyltransferase family protein, more preferably an amino acid sequence derived from a cis-prenyltransferase family protein.

The gene coding for the prenyltransferase family protein or the gene coding for the cis-prenyltransferase (CPT) family protein may be of any origin and may be of microbial, animal, or plant origin, preferably of plant origin. More preferably, it is derived from at least one selected from the group consisting of plants of the genera *Hevea*, *Sonchus*, *Taraxacum*, and *Parthenium*, still more preferably from a plant of the genus *Hevea* or *Taraxacum,* particularly preferably *Hevea brasiliensis* or *Taraxacum kok-saghyz*, most preferably *Hevea brasiliensis.*

Non-limiting examples of the plants include plants of the genus *Hevea* such as *Hevea brasiliensis*; plants of the genus *Sonchus* such as *Sonchus oleraceus*, *Sonchus asper*, and *Sonchus brachyotus*; plants of the genus *Solidago* such as *Solidago altissima*, *Solidago virgaurea* subsp. *asiatica*, *Solidago virgaurea* subsp. *leipcarpa*, *Solidago virgaurea* subsp. *leipcarpa* f. *paludosa*, *Solidago virgaurea* subsp. *gigantea*, and *Solidago gigantea* Ait. var. *leiophylla* Fernald; plants of the genus *Helianthus* such as *Helianthus annuus*, *Helianthus argophyllus*, *Helianthus atrorubens*, *Helianthus debilis*, *Helianthus decapetalus,* and *Helianthus giganteus*; plants of the genus *Taraxacum* such as dandelion (*Taraxacum), Taraxacum venustum* H. Koidz, *Taraxacum hondoense* Nakai, *Taraxacum platycarpum* Dahlst, *Taraxacum japonicum*, *Taraxacum officinale* Weber, *Taraxacum kok-saghyz,* and *Taraxacum brevicorniculatum;* plants of the genus *Ficus* such as *Ficus carica, Ficus elastica, Ficus pumila* L., *Ficus erecta* Thumb., *Ficus ampelas* Burm. f., *Ficus benguetensis* Merr., *Ficus irisana* Elm., *Ficus microcarpa* L. f., *Ficus septica* Burm. f., and *Ficus benghalensis;* plants of the genus *Parthenium* such as *Parthenium argentatum, Parthenium hysterophorus,* and annual ragweed (*Ambrosia artemisiifolia*)*;* lettuce (*Lactuca sativa*), *Ficus benghalensis,* and *Arabidopsis thaliana.*

As used herein, the term "prenyltransferase (CPT) family protein" refers to an enzyme that catalyzes a reaction of chain elongation of an isoprenoid compound.

Also as used herein, the term "trans-prenyltransferase (CPT) family protein" refers to an enzyme that catalyzes a reaction of trans-chain elongation of an isoprenoid compound.

Also as used herein, the term "cis-prenyltransferase (CPT) family protein" refers to an enzyme that catalyzes a reaction of cis-chain elongation of an isoprenoid compound.

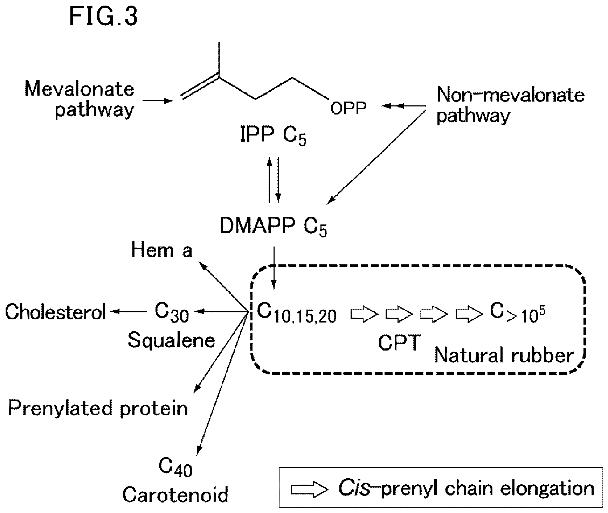

Specifically, in plants, for example, a polyisoprenoid is biosynthesized via a polyisoprenoid biosynthesis pathway as shown in Fig. 3, in which CPT family proteins are considered to be enzymes that catalyze the reactions enclosed by the dotted frame in Fig. 3. CPT family proteins are characterized by having an amino acid sequence contained in the cis-IPPS domain (NCBI accession No. cd00475).

As used herein, the term "isoprenoid compound" refers to a compound containing an isoprene unit (C₅H₈). Also, the term "cis-isoprenoid" refers to a compound including an isoprenoid compound in which isoprene units are cis-bonded, and examples include cis-farnesyl diphosphate, undecaprenyl diphosphate, and natural rubber.

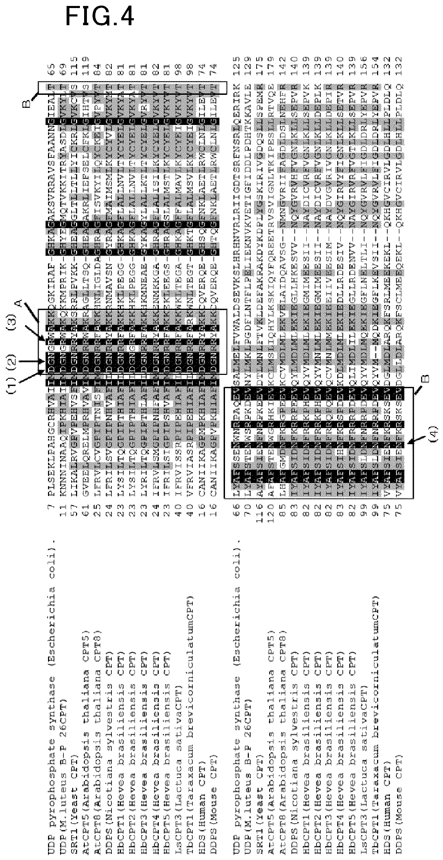

Here, Fig. 4 is an outline diagram showing the results of multiple sequence alignment of CPT family proteins derived from various organisms. According to literatures such as Shota Endo et.al., Biochimica et Biophysica Acta, No. 1625 (2003), pp. 291-295, and Masahiro Fujihashi et.al., PNAS, Vol. 98, No. 8 (2001), pp. 4337-4342, the box A (corresponding to positions 41 to 49 of HRT1 from *Hevea brasiliensis* represented by SEQ ID NO:2) and the box B (corresponding to positions 81 to 97 of HRT1 from *Hevea brasiliensis* represented by SEQ ID NO:2) in Fig. 4 are part of highly conserved regions between the CPT family proteins derived from various organisms. The term "conserved region" refers to a site having a similar sequence (structure) which is presumed to have a similar protein function. In particular, an aspartic acid residue conserved at a position corresponding to position 41 of HRT1 from *Hevea brasiliensis* represented by SEQ ID NO:2 ((1) in Fig. 4), a glycine residue conserved at a position corresponding to position 42 of HRT1 from *Hevea brasiliensis* represented by SEQ ID NO:2 ((2) in Fig. 4), an arginine residue conserved at a position corresponding to position 45 of HRT1 from *Hevea brasiliensis* represented by SEQ ID NO:2 ((3) in Fig. 4), and an asparagine residue conserved at a position corresponding to position 89 of HRT1 from *Hevea brasiliensis* represented by SEQ ID NO:2 ((4) in Fig. 4) are essential amino acids for the enzymatic reactions of CPT family proteins, and thus proteins having these amino acids at the respective positions are considered to have the functions of CPT family proteins.

The multiple sequence alignment can be performed as described in WO 2017/002818.

Thus, the CPT family protein preferably has an aspartic acid residue at position 41 of HRT1 from *Hevea brasiliensis* represented by SEQ ID NO:2 or at a corresponding position, a glycine residue at position 42 of HRT1 from *Hevea brasiliensis* represented by SEQ ID NO:2 or at a corresponding position, an arginine residue at position 45 of HRT1 from *Hevea brasiliensis* represented by SEQ ID NO:2 or at a corresponding position, and an asparagine residue at position 89 of HRT1 from *Hevea brasiliensis* represented by SEQ ID NO:2 or at a corresponding position. As described above, the CPT family protein having such a sequence is considered to have the functions of CPT family proteins and can function as an enzyme that catalyzes a reaction of cis-chain elongation of an isoprenoid compound.

More preferably, the CPT family protein has, at positions 41 to 49 of HRT1 from *Hevea brasiliensis* represented by SEQ ID NO:2 or at corresponding positions, the following amino acid sequence (A):

DGNX₁RX₂AKK (A)

wherein X₁ and X₂ are the same as or different from each other and each represent any amino acid residue, or an amino acid sequence that is identical to the amino acid sequence (A) regarding at least five out of the seven amino acid residues other than X₁ and X₂. Still more preferably, in the amino acid sequence (A), X₁ represents H, G, or R, and X₂ represents W, F, or Y.

The amino acid sequence that is identical to the amino acid sequence (A) regarding at least five out of the seven amino acid residues other than X₁ and X₂ is more preferably an amino acid sequence that is identical regarding at least six out of the seven amino acid residues other than X₁ and X₂.

Also more preferably, the CPT family protein has, at positions 81 to 97 of HRT1 from *Hevea brasiliensis* represented by SEQ ID NO:2 or at corresponding positions, the following amino acid sequence (B):

TX₁₁X₁₂AFSX₁₃X₁₄NX₁₅X₁₆RX₁₇X₁₈X₁₉EV (B)

wherein X₁₁ to X₁₉ are the same as or different from each other and each represent any amino acid residue, or an amino acid sequence that is identical to the amino acid sequence (B) regarding at least five out of the eight amino acid residues other than X₁₁ to X₁₉.

Still more preferably, in the amino acid sequence (B), X₁₁ represents L, V, A, or I; X₁₂ represents Y, F, or H; X₁₃ represents S, T, I, M, or L; X₁₄ represents E, D, or H; X₁₅ represents W or F; X₁₆ represents N, S, K, G, or R; X₁₇ represents P, S, H, G, R, K, or Q; X₁₈ represents A, K, S, or P; and X₁₉ represents Q, D, R, I, E, H, or S.

The amino acid sequence that is identical to the amino acid sequence (B) regarding at least five out of the eight amino acid residues other than X₁₁ to X₁₉ is more preferably an amino acid sequence that is identical regarding at least six, still more preferably at least seven, out of the eight amino acid residues other than X₁₁ to X₁₉.

Further, the CPT family protein particularly preferably has, at positions 41 to 49 of HRT1 from *Hevea brasiliensis* represented by SEQ ID NO:2 or at corresponding positions, an amino acid sequence that is identical to the amino acid sequence at positions 41 to 49 (DGNRRFAKK) of HRT1 from *Hevea brasiliensis* represented by SEQ ID NO:2 regarding at least six out of the nine amino acid residues. The amino acid sequence is more preferably identical regarding at least seven, still more preferably at least eight, out of the nine amino acid residues.

Further, the CPT family protein particularly preferably has, at positions 81 to 97 of HRT1 from *Hevea brasiliensis* represented by SEQ ID NO:2 or at corresponding positions, an amino acid sequence that is identical to the amino acid sequence at positions 81 to 97 (TIYAFSIDNFRRKPHEV) of HRT1 from *Hevea brasiliensis* represented by SEQ ID NO:2 regarding at least 14 out of the 17 amino acid residues. The amino acid sequence is more preferably identical regarding at least 15, still more preferably at least 16, out of the 17 amino acid residues.

Specifically, the conserved region corresponding to positions 41 to 49 of HRT1 from *Hevea brasiliensis* represented by SEQ ID NO:2 corresponds to, for example:
positions 25 to 33 of undecaprenyl pyrophosphate synthase (UPPS) from *Escherichia coli* represented by SEQ ID NO:45 disclosed in WO 2017/002818;
positions 29 to 37 of undecaprenyl diphosphate synthase (UPS) from *Micrococcus* represented by SEQ ID NO:46 disclosed in WO 2017/002818;
positions 75 to 83 of SRT1 from yeast represented by SEQ ID NO:47 disclosed in WO 2017/002818;
positions 79 to 87 of AtCPT5 from *Arabidopsis thaliana* represented by SEQ ID NO:44 disclosed in WO 2017/002818;
positions 43 to 51 of AtCPT8 from *Arabidopsis thaliana* represented by SEQ ID NO:22 disclosed in WO 2017/002818;
positions 42 to 50 of DDPS from tobacco represented by SEQ ID NO:48 disclosed in WO 2017/002818;
positions 41 to 49 of HRT2 from *Hevea brasiliensis* represented by SEQ ID NO:32 disclosed in WO 2017/002818;
positions 41 to 49 of CPT3 from *Hevea brasiliensis* represented by SEQ ID NO:36 disclosed in WO 2017/002818;
positions 42 to 50 of CPT4 from *Hevea brasiliensis* represented by SEQ ID NO:37 disclosed in WO 2017/002818;
positions 41 to 49 of CPT5 from *Hevea brasiliensis* represented by SEQ ID NO:41 disclosed in WO 2017/002818;
positions 58 to 66 of LsCPT3 from lettuce represented by SEQ ID NO:14 disclosed in WO 2017/002818;
positions 58 to 66 of TbCPT1 from *Taraxacum brevicorniculatum* represented by SEQ ID NO:43 disclosed in WO 2017/002818;
positions 34 to 42 of DDPS from mouse represented by SEQ ID NO:49 disclosed in WO 2017/002818; and
positions 34 to 42 of HDS from human represented by SEQ ID NO:50 disclosed in WO 2017/002818.

Moreover, the conserved region corresponding to positions 81 to 97 of HRT1 from *Hevea brasiliensis* represented by SEQ ID NO:2 corresponds to, for example:
positions 65 to 81 of undecaprenyl pyrophosphate synthase (UPPS) from *Escherichia coli* represented by SEQ ID NO:45 disclosed in WO 2017/002818;
positions 69 to 85 of undecaprenyl diphosphate synthase (UPS) from *Micrococcus* represented by SEQ ID NO:46 disclosed in WO 2017/002818;
positions 115 to 131 of SRT1 from yeast represented by SEQ ID NO:47 disclosed in WO 2017/002818;
positions 119 to 135 of AtCPT5 from *Arabidopsis thaliana* represented by SEQ ID NO:44 disclosed in WO 2017/002818;
positions 84 to 100 of AtCPT8 from *Arabidopsis thaliana* represented by SEQ ID NO:22 disclosed in WO 2017/002818;
positions 82 to 98 of DDPS from tobacco represented by SEQ ID NO:48 disclosed in WO 2017/002818;
positions 81 to 97 of HRT2 from *Hevea brasiliensis* represented by SEQ ID NO:32 disclosed in WO 2017/002818;
positions 81 to 97 of CPT3 from *Hevea brasiliensis* represented by SEQ ID NO:36 disclosed in WO 2017/002818;
positions 82 to 98 of CPT4 from *Hevea brasiliensis* represented by SEQ ID NO:37 disclosed in WO 2017/002818;
positions 81 to 97 of CPT5 from *Hevea brasiliensis* represented by SEQ ID NO:41 disclosed in WO 2017/002818;
positions 98 to 114 of LsCPT3 from lettuce represented by SEQ ID NO:14 disclosed in WO 2017/002818;
positions 98 to 114 of TbCPT1 from *Taraxacum brevicorniculatum* represented by SEQ ID NO:43 disclosed in WO 2017/002818;
positions 74 to 90 of DDPS from mouse represented by SEQ ID NO:49 disclosed in WO 2017/002818; and
positions 74 to 90 of HDS from human represented by SEQ ID NO:50 disclosed in WO 2017/002818.

Moreover, the aspartic acid residue corresponding to position 41 of HRT1 from *Hevea brasiliensis* represented by SEQ ID NO:2 corresponds to, for example:
the aspartic acid residue at position 25 of undecaprenyl pyrophosphate synthase (UPPS) from *Escherichia coli* represented by SEQ ID NO:45 disclosed in WO 2017/002818;
the aspartic acid residue at position 29 of undecaprenyl diphosphate synthase (UPS) from *Micrococcus* represented by SEQ ID NO:46 disclosed in WO 2017/002818;
the aspartic acid residue at position 75 of SRT1 from yeast represented by SEQ ID NO:47 disclosed in WO 2017/002818;
the aspartic acid residue at position 79 of AtCPT5 from *Arabidopsis thaliana* represented by SEQ ID NO:44 disclosed in WO 2017/002818;
the aspartic acid residue at position 43 of AtCPT8 from *Arabidopsis thaliana* represented by SEQ ID NO:22 disclosed in WO 2017/002818;
the aspartic acid residue at position 42 of DDPS from tobacco represented by SEQ ID NO:48 disclosed in WO 2017/002818;
the aspartic acid residue at position 41 of HRT2 from *Hevea brasiliensis* represented by SEQ ID NO:32 disclosed in WO 2017/002818;
the aspartic acid residue at position 41 of CPT3 from *Hevea brasiliensis* represented by SEQ ID NO:36 disclosed in WO 2017/002818;
the aspartic acid residue at position 42 of CPT4 from *Hevea brasiliensis* represented by SEQ ID NO:37 disclosed in WO 2017/002818;
the aspartic acid residue at position 41 of CPT5 from *Hevea brasiliensis* represented by SEQ ID NO:41 disclosed in WO 2017/002818;
the aspartic acid residue at position 58 of LsCPT3 from lettuce represented by SEQ ID NO:14 disclosed in WO 2017/002818;
the aspartic acid residue at position 58 of TbCPT1 from *Taraxacum brevicorniculatum* represented by SEQ ID NO:43 disclosed in WO 2017/002818;
the aspartic acid residue at position 34 of DDPS from mouse represented by SEQ ID NO:49 disclosed in WO 2017/002818; and
the aspartic acid residue at position 34 of HDS from human represented by SEQ ID NO:50 disclosed in WO 2017/002818.

Moreover, the glycine residue corresponding to position 42 of HRT1 from *Hevea brasiliensis* represented by SEQ ID NO:2 corresponds to, for example:
the glycine residue at position 26 of undecaprenyl pyrophosphate synthase (UPPS) from *Escherichia coli* represented by SEQ ID NO:45 disclosed in WO 2017/002818;
the glycine residue at position 30 of undecaprenyl diphosphate synthase (UPS) from *Micrococcus* represented by SEQ ID NO:46 disclosed in WO 2017/002818;
the glycine residue at position 76 of SRT1 from yeast represented by SEQ ID NO:47 disclosed in WO 2017/002818;
the glycine residue at position 80 of AtCPT5 from *Arabidopsis thaliana* represented by SEQ ID NO:44 disclosed in WO 2017/002818;
the glycine residue at position 44 of AtCPT8 from *Arabidopsis thaliana* represented by SEQ ID NO:22 disclosed in WO 2017/002818;
the glycine residue at position 43 of DDPS from tobacco represented by SEQ ID NO:48 disclosed in WO 2017/002818;
the glycine residue at position 42 of HRT2 from *Hevea brasiliensis* represented by SEQ ID NO:32 disclosed in WO 2017/002818;
the glycine residue at position 42 of CPT3 from *Hevea brasiliensis* represented by SEQ ID NO:36 disclosed in WO 2017/002818;
the glycine residue at position 43 of CPT4 from *Hevea brasiliensis* represented by SEQ ID NO:37 disclosed in WO 2017/002818;
the glycine residue at position 42 of CPT5 from *Hevea brasiliensis* represented by SEQ ID NO:41 disclosed in WO 2017/002818;
the glycine residue at position 59 of LsCPT3 from lettuce represented by SEQ ID NO:14 disclosed in WO 2017/002818;
the glycine residue at position 59 of TbCPT1 from *Taraxacum brevicorniculatum* represented by SEQ ID NO:43 disclosed in WO 2017/002818;
the glycine residue at position 35 of DDPS from mouse represented by SEQ ID NO:49 disclosed in WO 2017/002818; and
the glycine residue at position 35 of HDS from human represented by SEQ ID NO:50 disclosed in WO 2017/002818.

Moreover, the arginine residue corresponding to position 45 of HRT1 from *Hevea brasiliensis* represented by SEQ ID NO:2 corresponds to, for example:
the arginine residue at position 29 of undecaprenyl pyrophosphate synthase (UPPS) from *Escherichia coli* represented by SEQ ID NO:45 disclosed in WO 2017/002818;
the arginine residue at position 33 of undecaprenyl diphosphate synthase (UPS) from *Micrococcus* represented by SEQ ID NO:46 disclosed in WO 2017/002818;
the arginine residue at position 79 of SRT1 from yeast represented by SEQ ID NO:47 disclosed in WO 2017/002818;
the arginine residue at position 83 of AtCPT5 from *Arabidopsis thaliana* represented by SEQ ID NO:44 disclosed in WO 2017/002818;
the arginine residue at position 47 of AtCPT8 from *Arabidopsis thaliana* represented by SEQ ID NO:22 disclosed in WO 2017/002818;
the arginine residue at position 46 of DDPS from tobacco represented by SEQ ID NO:48 disclosed in WO 2017/002818;
the arginine residue at position 45 of HRT2 from *Hevea brasiliensis* represented by SEQ ID NO:32 disclosed in WO 2017/002818;
the arginine residue at position 45 of CPT3 from *Hevea brasiliensis* represented by SEQ ID NO:36 disclosed in WO 2017/002818;
the arginine residue at position 46 of CPT4 from *Hevea brasiliensis* represented by SEQ ID NO:37 disclosed in WO 2017/002818;
the arginine residue at position 45 of CPT5 from *Hevea brasiliensis* represented by SEQ ID NO:41 disclosed in WO 2017/002818;
the arginine residue at position 62 of LsCPT3 from lettuce represented by SEQ ID NO:14 disclosed in WO 2017/002818;
the arginine residue at position 62 of TbCPT1 from *Taraxacum brevicorniculatum* represented by SEQ ID NO:43 disclosed in WO 2017/002818;
the arginine residue at position 38 of DDPS from mouse represented by SEQ ID NO:49 disclosed in WO 2017/002818; and
the arginine residue at position 38 of HDS from human represented by SEQ ID NO:50 disclosed in WO 2017/002818.

Moreover, the asparagine residue corresponding to position 89 of HRT1 from *Hevea brasiliensis* represented by SEQ ID NO:2 corresponds to, for example:
the asparagine residue at position 73 of undecaprenyl pyrophosphate synthase (UPPS) from *Escherichia coli* represented by SEQ ID NO:45 disclosed in WO 2017/002818;
the asparagine residue at position 77 of undecaprenyl diphosphate synthase (UPS) from *Micrococcus* represented by SEQ ID NO:46 disclosed in WO 2017/002818;
the asparagine residue at position 123 of SRT1 from yeast represented by SEQ ID NO:47 disclosed in WO 2017/002818;
the asparagine residue at position 127 of AtCPT5 from *Arabidopsis thaliana* represented by SEQ ID NO:44 disclosed in WO 2017/002818;
the asparagine residue at position 92 of AtCPT8 from *Arabidopsis thaliana* represented by SEQ ID NO:22 disclosed in WO 2017/002818;
the asparagine residue at position 90 of DDPS from tobacco represented by SEQ ID NO:48 disclosed in WO 2017/002818;
the asparagine residue at position 89 of HRT2 from *Hevea brasiliensis* represented by SEQ ID NO:32 disclosed in WO 2017/002818;
the asparagine residue at position 89 of CPT3 from *Hevea brasiliensis* represented by SEQ ID NO:36 disclosed in WO 2017/002818;
the asparagine residue at position 90 of CPT4 from *Hevea brasiliensis* represented by SEQ ID NO:37 disclosed in WO 2017/002818;
the asparagine residue at position 89 of CPT5 from *Hevea brasiliensis* represented by SEQ ID NO:41 disclosed in WO 2017/002818;
the asparagine residue at position 106 of LsCPT3 from lettuce represented by SEQ ID NO:14 disclosed in WO 2017/002818;
the asparagine residue at position 106 of TbCPT1 from *Taraxacum brevicorniculatum* represented by SEQ ID NO:43 disclosed in WO 2017/002818;
the asparagine residue at position 82 of DDPS from mouse represented by SEQ ID NO:49 disclosed in WO 2017/002818; and
the asparagine residue at position 82 of HDS from human represented by SEQ ID NO:50 disclosed in WO 2017/002818.

Examples of the CPT family protein include CPT from *Hevea brasiliensis* (HRT1, HRT2, CPT3 to CPT5), AtCPT1 to AtCPT9 from *Arabidopsis thaliana,* CPT1 to CPT3 from lettuce, CPT1 to CPT3 from *Taraxacum brevicorniculatum,* undecaprenyl pyrophosphate synthase (UPPS) from *Escherichia coli,* undecaprenyl diphosphate synthase (UPS) from *Micrococcus,* SRT1 from yeast, DDPS from tobacco, DDPS from mouse, and HDS from human.

Specific examples of the CPT family protein include the following protein [11]:
a protein having the amino acid sequence represented by SEQ ID NO:2.

Moreover, it is known that proteins having one or more amino acid substitutions, deletions, insertions, or additions relative to the original amino acid sequence can have the inherent function. Thus, another specific example of the CPT family protein is the following protein [12]:
a protein having an amino acid sequence containing one or more amino acid substitutions, deletions, insertions, and/or additions relative to the amino acid sequence represented by SEQ ID NO:2, and having an enzymatic activity that catalyzes a reaction of cis-chain elongation of an isoprenoid compound.

To maintain the function of the CPT family protein, the protein preferably has an amino acid sequence containing one or more, more preferably 1 to 58, still more preferably 1 to 44, further preferably 1 to 29, particularly preferably 1 to 15, most preferably 1 to 6, still most preferably 1 to 3 amino acid substitutions, deletions, insertions, and/or additions relative to the amino acid sequence represented by SEQ ID NO:2.

Herein, preferred examples of amino acid substitutions are conservative substitutions. Specific examples include substitutions within each of the following groups in the parentheses: (glycine, alanine), (valine, isoleucine, leucine), (aspartic acid, glutamic acid), (asparagine, glutamine), (serine, threonine), (lysine, arginine), and (phenylalanine, tyrosine).

It is also known that proteins with amino acid sequences having high sequence identity to the original amino acid sequence can also have similar functions. Thus, another specific example of the CPT family protein is the following protein [13]:
a protein having an amino acid sequence with at least 80% sequence identity to the amino acid sequence represented by SEQ ID NO:2, and having an enzymatic activity that catalyzes a reaction of cis-chain elongation of an isoprenoid compound.

To maintain the function of the CPT family protein, the sequence identity to the amino acid sequence represented by SEQ ID NO:2 is preferably at least 85%, more preferably at least 90%, still more preferably at least 95%, particularly preferably at least 98%, most preferably at least 99%.

Specific examples of the CPT family protein also include the following proteins [14] to [16].
[14] a protein having the amino acid sequence represented by SEQ ID NO:3
[15] a protein having an amino acid sequence containing one or more amino acid substitutions, deletions, insertions, and/or additions relative to the amino acid sequence represented by SEQ ID NO:3, and having an enzymatic activity that catalyzes a reaction of cis-chain elongation of an isoprenoid compound

To maintain the function of the CPT family protein, the protein preferably has an amino acid sequence containing one or more, more preferably 1 to 60, still more preferably 1 to 45, particularly preferably 1 to 30, most preferably 1 to 15, still most preferably 1 to 6, further most preferably 1 to 3 amino acid substitutions, deletions, insertions, and/or additions relative to the amino acid sequence represented by SEQ ID NO:3.

[16] a protein having an amino acid sequence with at least 80% sequence identity to the amino acid sequence represented by SEQ ID NO:3, and having an enzymatic activity that catalyzes a reaction of cis-chain elongation of an isoprenoid compound

To maintain the function of the CPT family protein, the sequence identity to the amino acid sequence represented by SEQ ID NO:3 is preferably at least 85%, more preferably at least 90%, still more preferably at least 95%, particularly preferably at least 98%, most preferably at least 99%.

Herein, the sequence identity between amino acid sequences or between nucleotide sequences may be determined using the algorithm BLAST [Pro. Natl. Acad. Sci. USA, 90, 5873 (1993)] developed by Karlin and Altschul or FASTA [Methods Enzymol., 183, 63 (1990)].

Whether it is a protein having the above enzymatic activity may be determined by, for example, conventional techniques, such as by expressing a target protein in a transformant produced by introducing a gene coding for the target protein into *Escherichia coli* or other host organisms, and determining the presence or absence of the function of the target protein by the corresponding activity measuring method.

The gene coding for a protein having the second amino acid sequence may be any gene that codes for the protein having the second amino acid sequence to express and produce the protein having the second amino acid sequence.

The gene coding for the CPT family protein may be any gene that codes for the CPT family protein to express and produce the CPT family protein. Specific examples of the gene include the following DNAs [11] and [12]:
[11] a DNA having the nucleotide sequence represented by SEQ ID NO:1; and
[12] a DNA which hybridizes under stringent conditions to a DNA having a nucleotide sequence complementary to the nucleotide sequence represented by SEQ ID NO:1, and which codes for a protein having an enzymatic activity that catalyzes a reaction of cis-chain elongation of an isoprenoid compound.

As used herein, the term "hybridize" means a process in which a DNA hybridizes to a DNA having a specific nucleotide sequence or a part of the DNA. Thus, the DNA having a specific nucleotide sequence or part of the DNA may have a nucleotide sequence long enough to be usable as a probe in Northern or Southern blot analysis or as an oligonucleotide primer in polymerase chain reaction (PCR) analysis. The DNA used as a probe may have a length of at least 100 bases, preferably at least 200 bases, more preferably at least 500 bases, but it may be a DNA of at least 10 bases, preferably of at least 15 bases in length.

Techniques to perform DNA hybridization experiments are well known. The hybridization conditions under which experiments are carried out may be determined according to, for example, Molecular Cloning, 2nd ed. and 3rd ed. (2001), Methods for General and Molecular Bacteriology, ASM Press (1994), Immunology methods manual, Academic press (Molecular), and many other standard textbooks.

Herein, the stringent conditions may include, for example, an overnight incubation at 42°C of a DNA-immobilized filter and a DNA probe in a solution containing 50% formamide, 5× SSC (750 mM sodium chloride, 75 mM sodium citrate), 50 mM sodium phosphate (pH 7.6), 5× Denhardt's solution, 10% dextran sulfate, and 20 µg/L denatured salmon sperm DNA, followed by washing the filter for example in a 0.2× SSC solution at approximately 65°C. Less stringent conditions may also be used. Changes in the stringency may be accomplished by manipulating the formamide concentration (lower percentages of formamide result in lower stringency), salt concentrations, or temperature. For example, low stringent conditions include an overnight incubation at 37°C in a solution containing 6× SSCE (20× SSCE: 3 mol/L sodium chloride, 0.2 mol/L sodium dihydrogen phosphate, 0.02 mol/L EDTA, pH 7.4), 0.5% SDS, 30% formamide, and 100 µg/L denatured salmon sperm DNA, followed by washing in a 1× SSC solution containing 0.1% SDS at 50°C. In addition, to achieve even lower stringency, washes performed following hybridization may be done at higher salt concentrations (e.g., 5× SSC) in the above-mentioned low stringent conditions.

Variations in the above various conditions may be accomplished through the inclusion or substitution of blocking reagents used to suppress background in hybridization experiments. The inclusion of blocking reagents may require modification of the hybridization conditions for compatibility.

The DNA capable of hybridizing under stringent conditions as described above may have a nucleotide sequence with at least 80%, preferably at least 90%, more preferably at least 95%, still more preferably at least 98%, particularly preferably at least 99% sequence identity to the nucleotide sequence represented by SEQ ID NO:1 as calculated using a program such as BLAST or FASTA with the parameters mentioned above.

Whether the DNA which hybridizes to the above-mentioned DNA under stringent conditions codes for a protein having a predetermined enzymatic activity may be determined by conventional techniques, such as by expressing a target protein in a transformant produced by introducing a gene coding for the target protein into *Escherichia coli* or other host organisms, and determining the presence or absence of the function of the target protein by the corresponding activity measuring method.

Moreover, conventional techniques may be employed to identify the amino acid sequence or nucleotide sequence of the proteins. For example, total RNA is extracted from a growing plant, the mRNA is optionally purified, and a cDNA is synthesized by a reverse transcription reaction. Subsequently, degenerate primers are designed based on the amino acid sequence of a known protein corresponding to the target protein, a DNA fragment is partially amplified by RT-PCR, and the sequence is partially identified. Then, the RACE method or the like is performed to identify the full-length nucleotide sequence or amino acid sequence. The RACE method (rapid amplification of cDNA ends method) refers to a method in which, when the nucleotide sequence of a cDNA is partially known, PCR is performed based on the nucleotide sequence information of such a known region to clone an unknown region extending to the cDNA terminal. This method can clone full-length cDNA by PCR without preparing a cDNA library.

The degenerate primers may preferably be prepared from plant-derived sequences having a highly similar sequence part to the target protein.

Moreover, if the nucleotide sequence coding for the protein is known, the full-length nucleotide sequence or amino acid sequence can be identified by designing a primer containing a start codon and a primer containing a stop codon using the known nucleotide sequence, followed by performing RT-PCR using a synthesized cDNA as a template.

### (First amino acid sequence)

The first amino acid sequence is an amino acid sequence capable of binding to lipid droplets.

As used herein, binding of a protein to lipid droplets means, for example, but not limited to, that the protein is fully or partially incorporated into the lipid droplets or inserted into the membrane structure of the lipid droplets, and also means embodiments in which, for example, the protein localizes to the surface or inside of the lipid droplets. Moreover, the concept of binding to lipid droplets also includes embodiments in which the protein forms a complex with another protein bound to the lipid droplets to be present as the complex on the lipid droplets.

Although the amino acid sequence capable of binding to lipid droplets may be any amino acid sequence capable of binding to lipid droplets, it is preferably an amino acid sequence derived from a protein capable of binding to lipid droplets, more preferably an amino acid sequence derived from a protein capable of binding to lipid droplets which is a class II protein. Here, since it is sufficient that the amino acid sequence derived from a protein capable of binding to lipid droplets is capable of binding to lipid droplets, it may be the entire amino acid sequence of the protein capable of binding to lipid droplets or a part of the amino acid sequence. The amino acid sequence capable of binding to lipid droplets is not limited, but is desirably an amino acid sequence of a transmembrane domain of a protein that is naturally bound to lipid droplets in the natural world.

As used herein, class II proteins refer to lipid droplet-binding proteins characterized by being capable of binding to lipid droplets, and further by localizing to the cytosol when lipid droplets are absent in cells (c.f., Gidda et al., Plant Physiology!, April 2016, Vol. 170, pp. 2052-2071). Unlike class I proteins which localize to ER fractions when lipid droplets are absent, class II proteins can transfer between the cytosol and lipid droplets.

Non-limiting examples of the protein capable of binding to lipid droplets which is a class II protein include lipid droplet-associated protein (LDAP)/small rubber particle protein (SRPP) family proteins. Preferred are lipid droplet-associated protein (LDAP)/small rubber particle protein (SRPP) family proteins, among others.

Thus, the first amino acid sequence is preferably an amino acid sequence derived from a lipid droplet-associated protein (LDAP)/small rubber particle protein (SRPP) family protein.

The gene coding for the LDAP/SRPP family protein may be of any origin and may be of microbial, animal, or plant origin, preferably of plant origin.

Examples of the plant include plants as listed above, as well as plants of the genus *Persea* such as *Persea americana,* plants of the genus *Sesamum* such as *Sesamum indicum,* plants of the genus *Brassica* such as *Brassica napus,* and plants of the genus *Camellia* such as *Camellia japonica.*

The gene coding for the LDAP/SRPP family protein is preferably derived from a plant, more preferably from at least one selected from the group consisting of plants of the genera *Persea, Sesamum, Brassica, Camellia, Hevea, Sonchus, Taraxacum,* and *Parthenium,* still more preferably from at least one selected from the group consisting of plants of the genera *Persea, Hevea,* and *Taraxacum,* particularly preferably from at least one plant selected from the group consisting of *Persea americana, Hevea brasiliensis,* and *Taraxacum kok-saghyz,* most preferably from *Persea americana* or *Hevea brasiliensis.*

Thus, the first amino acid sequence is preferably an amino acid sequence derived from a LDAP/SRPP family protein of plant origin, more preferably an amino acid sequence derived from a LDAP/SRPP family protein derived from at least one selected from the group consisting of plants of the genera *Persea, Sesamum, Brassica, Camellia, Hevea, Sonchus, Taraxacum,* and *Parthenium,* still more preferably an amino acid sequence derived from a LDAP/SRPP family protein derived from at least one selected from the group consisting of plants of the genera *Persea, Hevea,* and *Taraxacum,* particularly preferably an amino acid sequence derived from a LDAP/SRPP family protein derived from at least one plant selected from the group consisting of *Persea americana, Hevea brasiliensis,* and *Taraxacum kok-saghyz,* most preferably an amino acid sequence derived from a LDAP/SRPP family protein derived from *Persea americana* or *Hevea brasiliensis.*

Examples of the LDAP/SRPP family protein include LDAP from *Persea americana* (LDAP1, LDAP2), SRPP or REF from *Hevea brasiliensis,* SRPP from *Taraxacum kok-saghyz,* and REF or SRPP from plants of the genus *Ficus.*

Specific examples of the LDAP/SRPP family protein include the following proteins [101] to [103]:
[101] a protein having the amino acid sequence represented by SEQ ID NO:4;
[102] a protein having the amino acid sequence represented by SEQ ID NO:10; and
[103] a protein having the amino acid sequence represented by SEQ ID NO:11.

It is also known that proteins with amino acid sequences having high sequence identity to the original amino acid sequence can also have similar functions. Thus, other specific examples of the LDAP/SRPP family protein include the following proteins [104] to [106]:
[104] a protein having an amino acid sequence with at least 80% sequence identity to the amino acid sequence represented by SEQ ID NO:4, and being capable of binding to lipid droplets;
[105] a protein having an amino acid sequence with at least 80% sequence identity to the amino acid sequence represented by SEQ ID NO:10, and being capable of binding to lipid droplets; and
[106] a protein having an amino acid sequence with at least 80% sequence identity to the amino acid sequence represented by SEQ ID NO:11, and being capable of binding to lipid droplets.

To maintain the function of the LDAP/SRPP family protein, the sequence identity to the amino acid sequence represented by SEQ ID NO:4, 10, or 11 is preferably at least 85%, more preferably at least 90%, still more preferably at least 95%, particularly preferably at least 98%, most preferably at least 99%.

Whether it is a protein capable of binding to lipid droplets may be determined by, for example, conventional techniques, such as by expressing a target protein in a transformant produced by introducing a gene coding for the target protein into *Escherichia coli* or other host organisms, and determining the presence or absence of the function of the target protein.

The gene coding for a protein having the first amino acid sequence may be any gene that codes for the protein having the first amino acid sequence to express and produce the protein having the first amino acid sequence.

### (Third amino acid sequence)

The third amino acid sequence is a linker sequence disposed between the first amino acid sequence and the second amino acid sequence. The presence of the linker sequence allows the fusion protein to maintain the lipid droplet-binding ability of the first amino acid sequence and the enzymatic activity of the second amino acid sequence. Moreover, the linker sequence is characterized by the number of amino acids constituting the sequence, not the types of amino acids constituting the sequence. Thus, the lipid droplet-binding ability of the first amino acid sequence and the enzymatic activity of the second amino acid sequence can be maintained when the first amino acid sequence and the second amino acid sequence are spaced apart by at least a predetermined distance.

Although the third amino acid sequence may be any sequence that can maintain the lipid droplet-binding ability of the first amino acid sequence and the enzymatic activity of the second amino acid sequence, it preferably consists of 4 or more amino acids, more preferably 8 or more amino acids, still more preferably 10 or more amino acids, particularly preferably 15 or more amino acids, most preferably 20 or more amino acids. The upper limit of the number of amino acids is not limited, but it preferably consists of 50 or less amino acids, more preferably 45 or less amino acids, still more preferably 40 or less amino acids, particularly preferably 35 or less amino acids, most preferably 30 or less amino acids.

The third amino acid sequence may be of any origin and may be of microbial, animal, or plant origin, preferably of plant origin. It may also be an artificially produced amino acid sequence.

Examples of the plant include plants as described for the origin of the gene coding for the LDAP/SRPP family protein.

Specific examples of the third amino acid sequence include the following amino acid sequences [1] to [3]. [1] the amino acid sequence represented by SEQ ID NO:5

[2] an amino acid sequence containing one or more amino acid substitutions, deletions, insertions, and/or additions relative to the amino acid sequence represented by SEQ ID NO:5

It preferably has an amino acid sequence containing one or more, more preferably 1 to 5, still more preferably 1 to 4, particularly preferably 1 to 3, most preferably 1 to 2, still most preferably 1 amino acid substitutions, deletions, insertions, and/or additions relative to the amino acid sequence represented by SEQ ID NO:5.

[3] an amino acid sequence with at least 80% sequence identity to the amino acid sequence represented by SEQ ID NO:5

The sequence identity to the amino acid sequence represented by SEQ ID NO:5 is preferably at least 85%, more preferably at least 90%, still more preferably at least 95%, particularly preferably at least 98%, most preferably at least 99%.

The gene coding for a protein having the third amino acid sequence may be any gene that codes for the protein having the third amino acid sequence to express and produce the protein having the third amino acid sequence.

### (Fusion protein)

The fusion protein of the present disclosure contains the first amino acid sequence and the second amino acid sequence which are fused via the third amino acid sequence. Moreover, the presence of the third amino acid sequence (linker sequence) allows the fusion protein to maintain the lipid droplet-binding ability of the first amino acid sequence and the enzymatic activity of the second amino acid sequence.

Once the sequences of the first to third amino acid sequences are determined, a person skilled in the art can produce the fusion protein by known techniques.

The method for producing the fusion protein preferably includes a gene preparation step involving linking DNA fragments of a gene coding for a protein having the first amino acid sequence, a gene coding for a protein having the second amino acid sequence, and a gene coding for a protein having the third amino acid sequence to prepare a gene coding for the fusion protein.

Moreover, when the fusion protein is produced by cell-free protein synthesis, the method preferably includes, in addition to the gene preparation step, preparing a mRNA based on the gene coding for the fusion protein, and performing protein synthesis using a cell-free protein synthesis solution containing the mRNA coding for the fusion protein to produce the fusion protein.

When the fusion protein is produced using a transgenic cell, the method preferably includes, in addition to the gene preparation step,
preparing a transgenic cell into which the gene coding for the fusion protein has been introduced, and
culturing the transgenic cell to produce the fusion protein.

The fusion protein may contain amino acid sequences other than the first amino acid sequence, the second amino acid sequence, and the third amino acid sequence. The proportion of the amino acids of the first, second, and third amino acid sequences relative to the total amino acids of the fusion protein is preferably 90% or higher, more preferably 95% or higher, still more preferably 98% or higher, particularly preferably 99% or higher.

### <Method for producing substance>

The method for producing a substance (hydrophobic compound) of the present disclosure includes binding the fusion protein to lipid droplets and accumulating a product (hydrophobic compound) in the lipid droplets by the enzymatic activity of the second amino acid sequence.

Since the fusion protein maintains the lipid droplet-binding ability of the first amino acid sequence and the enzymatic activity of the second amino acid sequence, the fusion protein can bind to lipid droplets and catalyze an enzymatic reaction on the lipid droplets by the enzymatic activity of the second amino acid sequence to produce a product (hydrophobic compound) which is accumulated in the lipid droplets.

As used herein, binding of the fusion protein to lipid droplets means, for example, but not limited to, that the fusion protein is fully or partially incorporated into the lipid droplets or inserted into the membrane structure of the lipid droplets, and also means embodiments in which, for example, the fusion protein localizes to the surface or inside of the lipid droplets. Moreover, the concept of binding to lipid droplets also includes embodiments in which the fusion protein forms a complex with another protein bound to the lipid droplets to be present as the complex on the lipid droplets.

As used herein, lipid droplets refer to inclusion bodies or cell organelles which have a membrane structure including phospholipids and membrane proteins (for example, oleosins or lipid droplet-associated protein (LDAP)/small rubber particle protein (SRPP) family proteins) and store triacylglycerols therein, as shown in Fig. 1. Lipid droplets exist in both prokaryotes and eukaryotes, including all plants.

Lipid droplets are also called oil droplets, fat droplets, oil body, oil bodies, or LD.

Although the lipid droplets may be of any origin, the lipid droplets are preferably of plant origin.

Examples of the plant include plants as described for the origin of the gene coding for the LDAP/SRPP family protein.

Among the plants, the lipid droplets are more preferably derived from at least one selected from the group consisting of plants of the genera *Persea, Sesamum, Brassica,* and *Camellia,* still more preferably from a plant of the genus *Persea,* particularly preferably from *Persea americana.* Moreover, the origins of the first amino acid sequence and the lipid droplets are preferably the same.

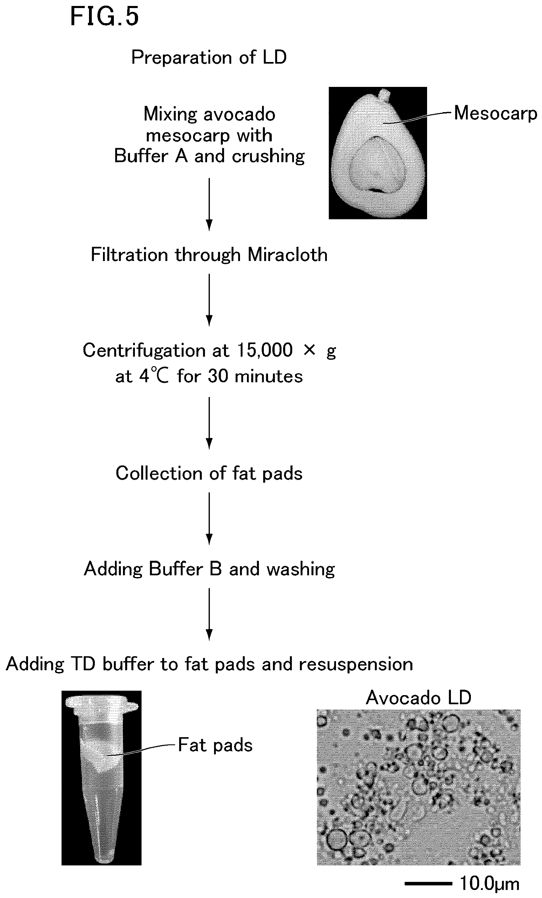

The lipid droplets may be prepared by any method including known methods. For example, a plant piece may be crushed in a buffer, followed by filtration and centrifugation to collect fat pads. The fat pads may also be washed, if necessary. Then, centrifugation may be performed to separate lipid droplets from the fat pads. Fig. 5 is a diagram showing a summary of an exemplary method for preparing lipid droplets.

The centrifugation may be carried out at 15,000 to 20,000 × g for 15 to 60 minutes.

Moreover, the centrifugation temperature is preferably 0 to 10°C, more preferably 2 to 8°C, particularly preferably 4°C.

The fusion protein may be bonded to lipid droplets by any method that can bind the fusion protein to the lipid droplets. An exemplary method includes performing protein synthesis in the presence of both a cell-free protein synthesis solution containing a mRNA coding for the fusion protein and lipid droplets to bind the fusion protein to the lipid droplets.

Thus, it is preferred to perform protein synthesis in the presence of both a cell-free protein synthesis solution containing a mRNA coding for the fusion protein and lipid droplets (more specifically, while mixing a cell-free protein synthesis solution containing a mRNA coding for the fusion protein with lipid droplets) to obtain the lipid droplets bound to the fusion protein.

Here, binding of the fusion protein to lipid droplets by protein synthesis in the presence of both the cell-free protein synthesis solution and the lipid droplets means, for example, but not limited to, that the fusion protein synthesized by the protein synthesis is fully or partially incorporated into the lipid droplets or inserted into the membrane structure of the lipid droplets, and also means embodiments in which, for example, the fusion protein localizes to the surface or inside of the lipid droplets. Moreover, the concept of binding to lipid droplets also includes embodiments in which the fusion protein forms a complex with another protein bound to the lipid droplets to be present as the complex on the lipid droplets as described above.

The mRNA coding for the fusion protein serves as a translation template that can be translated to synthesize the fusion protein. Moreover, the mRNA coding for the fusion protein may be prepared by any method as long as it serves as a translation template that can be translated to synthesize the fusion protein. For example, the mRNA may be prepared by determining a nucleotide sequence coding for the fusion protein based on the amino acid sequence information of the fusion protein, obtaining a DNA fragment of a gene coding for the fusion protein based on the determined nucleotide sequence information, and performing an ordinary in vitro transcription reaction of the DNA fragment.

Alternatively, the mRNA may be prepared by linking DNA fragments of a gene coding for a protein having the first amino acid sequence, a gene coding for a protein having the second amino acid sequence, and a gene coding for a protein having the third amino acid sequence, obtaining a DNA fragment of a gene coding for the fusion protein based on the nucleotide sequence information of the linked DNA fragments, and performing an ordinary in vitro transcription reaction of the DNA fragment.

As long as the cell-free protein synthesis solution contains the mRNA coding for the fusion protein, it may contain mRNAs coding for other proteins.

The mRNAs coding for other proteins may be those that can be translated to express the other proteins.

In addition to the mRNA (translation template) coding for the fusion protein, the cell-free protein synthesis solution preferably contains ATP, GTP, creatine phosphate, creatine kinase, L-amino acids, potassium ions, magnesium ions, and other components required for protein synthesis, as well as activity enhancers, if necessary. The use of such a cell-free protein synthesis solution allows for a cell-free protein synthesis reaction system.

Examples of reaction systems or apparatuses for protein synthesis that may be used in the cell-free protein synthesis include a batch method (Pratt, J.M. et al., Transcription and Translation, Hames, 179-209, B.D. & Higgins, S.J., eds, IRL Press, Oxford (1984)), a continuous cell-free protein synthesis system in which amino acids, energy sources, and other components are supplied continuously to the reaction system (Spirin, A.S. et al., Science, 242, 1162-1164 (1988)), a dialysis method (Kigawa et al., 21st Annual Meeting of the Molecular Biology Society of Japan, WID 6), and an overlay method (instruction manual of PROTEIOS^{™} wheat germ cell-free protein synthesis core kit, Toyobo Co., Ltd.). Other methods may include supplying template RNA, amino acids, energy sources, and other components as necessary to the protein synthesis reaction system, and discharging the synthesis product or decomposition product as required.

A person skilled in the art can perform the protein synthesis in the presence of both the cell-free protein synthesis solution and lipid droplets with reference to the method described in WO 2017/002818, for example.

The production method of the present disclosure may include, after binding the fusion protein to lipid droplets, collecting the lipid droplets bound to the fusion protein, if necessary.

The lipid droplet-collecting step may be carried out by any method that can collect the lipid droplets, including methods usually used to collect lipid droplets. Specific examples include methods involving centrifugation. When the lipid droplets are collected by such a centrifugation method, the centrifugal force, centrifugation time, and centrifugation temperature may be appropriately selected so as to be able to collect the lipid droplets.

The centrifugation may be performed, for example, at 15,000 to 20,000 × g for 15 to 60 minutes.

Moreover, from the standpoint of maintaining the activity of the fusion protein bound to lipid droplets, the centrifugation temperature is preferably 0 to 10°C, more preferably 2 to 8°C, particularly preferably 4°C.

For example, when the cell-free protein synthesis is performed, the centrifugation may be performed to separate the lipid droplets and the cell-free protein synthesis solution into the upper and lower layers, respectively. Then, the upper lipid droplet layer may be collected to collect the lipid droplets bound to the fusion protein. The collected lipid droplets may be re-suspended in an appropriate buffer with a neutral pH for storage.

Then, the hydrophobic compound (e.g., a polyisoprenoid) may be synthesized using the lipid droplets bound to the fusion protein. Specifically, the hydrophobic compound may be synthesized by adding a substrate corresponding to the enzymatic activity of the second amino acid sequence to the lipid droplets bound to the fusion protein to cause an enzymatic reaction. For example, when the second amino acid sequence used is an amino acid sequence derived from a cis-prenyltransferase family protein, isopentenyl diphosphate (IPP), optionally together with farnesyl diphosphate (FPP), may be added as a substrate to synthesize a polyisoprenoid (natural rubber). Then, the synthesized hydrophobic compound is accumulated in the lipid droplets.

As described above, the method for producing a substance (hydrophobic compound) of the present disclosure includes binding the fusion protein to lipid droplets and accumulating a product (hydrophobic compound) in the lipid droplets by the enzymatic activity of the second amino acid sequence.

Moreover, the hydrophobic compound (e.g., polyisoprenoid (natural rubber)) produced by the production method of the present disclosure may be collected, for example, by subjecting the lipid droplets to a solidification step as described below.

The solidification step may be carried out by any solidification method, such as by adding the lipid droplets to a solvent that does not dissolve the polyisoprenoid (natural rubber), such as ethanol, methanol, or acetone, or adding an acid to the lipid droplets. The rubber (natural rubber) can be recovered as solids from the lipid droplets by the solidification step. The recovered rubber (natural rubber) may be dried if necessary before use.

As used herein, the term "polyisoprenoid" is a collective term for polymers composed of isoprene units (C₅H₈). Examples of the polyisoprenoid include sesterterpenes (C₂₅), triterpenes (C₃₀), tetraterpenes (C₄₀), natural rubber, and other polymers. Also as used herein, the term "isoprenoid" refers to a compound containing an isoprene unit (C₅H₈), and conceptually includes polyisoprenoids.

The weight average molecular weight (Mw) of the polyisoprenoid is preferably 1,000 or more, more preferably 10,000 or more, still more preferably 100,000 or more, and the upper limit is not critical.

The weight average molecular weight (Mw) is determined by gel permeation chromatography (GPC) under the following conditions (1) to (7).
(1) Apparatus: HLC-820 available from Tosoh Corporation
(2) Separation column: GMH-XL available from Tosoh Corporation
(3) Measurement temperature: 40°C
(4) Carrier: tetrahydrofuran
(5) Flow rate: 0.6 mL/min
(6) Detector: differential refractometer, UV
(7) Molecular weight standards: polystyrene standards

Here, as a result of investigation, the present inventors have found that when the second amino acid sequence used is an amino acid sequence derived from a certain CPT family protein, sufficient enzymatic activity may not be achieved by binding the fusion protein to lipid droplets.

It has also been found that in this case, a coenzyme may further be added to enhance the enzymatic activity of the fusion protein.

Non-limiting examples of the coenzyme include Nogo-B receptor (NgBR) family proteins.

Nogo-B receptor (NgBR) family proteins are proteins having the function of binding to a membrane via one or more transmembrane domains on the N-terminal side of the proteins and interacting with a CPT family protein or other proteins on the C-terminal side thereof, and thus can assist the function of CPT family proteins by holding the CPT family proteins on the membrane. The NgBR family proteins are characterized by having a transmembrane domain on the N-terminal side and an amino acid sequence contained in the cis-IPPS superfamily domain (NCBI accession No. COG0020) on the C-terminal side.

The gene coding for the NgBR family protein used may be of any origin and may be of microbial, animal, or plant origin, preferably of plant origin. More preferably, it is derived from at least one selected from the group consisting of plants of the genera *Hevea, Sonchus, Taraxacum,* and *Parthenium,* still more preferably from a plant of the genus *Hevea* or *Taraxacum,* particularly preferably from *Hevea brasiliensis* or *Taraxacum kok-saghyz,* most preferably from *Hevea brasiliensis.*

Moreover, the origins of the gene coding for the second amino acid sequence and the gene coding for the NgBR family protein are preferably the same.

Examples of the NgBR family protein include NgBR from *Hevea brasiliensis* (HRBP), AtLEW1 from *Arabidopsis thaliana,* LsCPTL1 to LsCPTL2 from lettuce, and TbRTA from dandelion.

Specific examples of the NgBR family protein include the following protein [201]:
a protein having the amino acid sequence represented by SEQ ID NO:6.

Moreover, it is known that proteins having one or more amino acid substitutions, deletions, insertions, or additions relative to the original amino acid sequence can have the inherent function. Thus, another specific example of the NgBR family protein is the following protein [202]: [202] a protein having an amino acid sequence containing one or more amino acid substitutions, deletions, insertions, and/or additions relative to the amino acid sequence represented by SEQ ID NO:6, and having the function of binding to a membrane via one or more transmembrane domains on the N-terminal side of the protein and interacting with another protein on the C-terminal side thereof.

To maintain the function of the NgBR family protein, the protein preferably has an amino acid sequence containing one or more, more preferably 1 to 52, still more preferably 1 to 39, further preferably 1 to 26, particularly preferably 1 to 13, most preferably 1 to 6, still most preferably 1 to 3 amino acid substitutions, deletions, insertions, and/or additions relative to the amino acid sequence represented by SEQ ID NO:6.

As described above, it is also known that proteins with amino acid sequences having high sequence identity to the original amino acid sequence can also have similar functions. Thus, another specific example of the NgBR family protein is the following protein [203]:
a protein having an amino acid sequence with at least 80% sequence identity to the amino acid sequence represented by SEQ ID NO:6, and having the function of binding to a membrane via one or more transmembrane domains on the N-terminal side of the protein and interacting with another protein on the C-terminal side thereof

To maintain the function of the NgBR family protein, the sequence identity to the amino acid sequence represented by SEQ ID NO:6 is preferably at least 85%, more preferably at least 90%, still more preferably at least 95%, particularly preferably at least 98%, most preferably at least 99%.

Here, since it is sufficient that the coenzyme has the function of binding to a membrane via one or more transmembrane domains on the N-terminal side of the protein and interacting with a CPT family protein or other proteins on the C-terminal side thereof, it may be the entire NgBR family protein or a part of the protein.

To more enhance the enzymatic activity of the fusion protein, the coenzyme is preferably any one of the following proteins [0204] to [0206].
[204] a protein having the amino acid sequence at positions 1 to 81 of the amino acid sequence represented by SEQ ID NO:6
[205] a protein having an amino acid sequence containing one or more amino acid substitutions, deletions, insertions, and/or additions relative to the amino acid sequence at positions 1 to 81 of the amino acid sequence represented by SEQ ID NO:6, and having the function of binding to a membrane via one or more transmembrane domains on the N-terminal side of the protein and interacting with another protein on the C-terminal side thereof
To maintain the function, the protein preferably has an amino acid sequence containing one or more, more preferably 1 to 16, still more preferably 1 to 12, particularly preferably 1 to 8, most preferably 1 to 4, still most preferably 1 to 2, further most preferably 1 amino acid substitutions, deletions, insertions, and/or additions relative to the amino acid sequence at positions 1 to 81 of the amino acid sequence represented by SEQ ID NO:6.
[206] a protein having an amino acid sequence with at least 80% sequence identity to the amino acid sequence at positions 1 to 81 of the amino acid sequence represented by SEQ ID NO:6, and having the function of binding to a membrane via one or more transmembrane domains on the N-terminal side of the protein and interacting with another protein on the C-terminal side thereof

To maintain the function, the sequence identity to the amino acid sequence at positions 1 to 81 of the amino acid sequence represented by SEQ ID NO:6 is preferably at least 85%, more preferably at least 90%, still more preferably at least 95%, particularly preferably at least 98%, most preferably at least 99%.

Whether it is a NgBR family protein described above may be determined by conventional techniques, such as by identifying the amino acid sequence and determining whether it has an amino acid sequence contained in the cis-IPPS superfamily domain (NCBI accession No. COG0020).

The gene coding for the NgBR family protein may be any gene that codes for the NgBR family protein to express and produce the NgBR family protein.

### <Method for producing rubber product>

The method for producing a rubber product of the present disclosure includes: producing a polyisoprenoid by the method for producing a substance (using droplets bound to the fusion protein); kneading the polyisoprenoid with an additive to obtain a kneaded mixture; forming a raw rubber product from the kneaded mixture; and vulcanizing the raw rubber product.

The rubber product may be any rubber product producible from rubber, preferably natural rubber. Examples include pneumatic tires, rubber rollers, rubber fenders, gloves, and medical rubber tubes.

When the rubber product is a pneumatic tire or, in other words, when the method for producing a rubber product of the present disclosure is the method for producing a pneumatic tire of the present disclosure, the raw rubber product forming step corresponds to forming a green tire from the kneaded mixture, and the vulcanization step corresponds to vulcanizing the green tire. Thus, the method for producing a pneumatic tire of the present disclosure includes: producing a polyisoprenoid as described above; kneading the polyisoprenoid with an additive to obtain a kneaded mixture; forming a green tire from the kneaded mixture; and vulcanizing the green tire.

### <Kneading step>

In the kneading step, the polyisoprenoid produced by the production method (using droplets bound to the fusion protein) is kneaded with an additive to obtain a kneaded mixture.

Any additive may be used, including additives used in the production of rubber products. For example, in the case where the rubber product is a pneumatic tire, examples of the additive include rubber components other than the polyisoprenoid, reinforcing fillers such as carbon black, silica, calcium carbonate, alumina, clay, and talc, silane coupling agents, zinc oxide, stearic acid, processing aids, various antioxidants, softeners such as oils, waxes, vulcanizing agents such as sulfur, and vulcanization accelerators.

In the kneading step, kneading may be carried out using an open roll mill, a Banbury mixer, an internal mixer, or other rubber kneading machines.

### (Raw rubber product forming step (green tire forming step in the case of tire))

In the raw rubber product forming step, a raw rubber product (green tire in the case of tire) is formed from the kneaded mixture obtained in the kneading step.

The raw rubber product may be formed by any method, including appropriate methods used to form raw rubber products. For example, in the case where the rubber product is a pneumatic tire, the kneaded mixture obtained in the kneading step may be extruded into the shape of a tire component and then formed and assembled with other tire components in a usual manner on a tire building machine to form a green tire (unvulcanized tire).

### (Vulcanization step)

In the vulcanization step, the raw rubber product obtained in the raw rubber product forming step is vulcanized to obtain a rubber product.

The raw rubber product may be vulcanized by any method, including appropriate methods used to vulcanize raw rubber products. For example, in the case where the rubber product is a pneumatic tire, the green tire (unvulcanized tire) obtained in the raw rubber product forming step may be vulcanized by heating and pressing in a vulcanizer to obtain a pneumatic tire.

### <Vector>

The vector of the present disclosure is a vector into which a gene coding for the fusion protein has been introduced.

By introducing such a vector into cells for transformation, the gene coding for the fusion protein in the vector can be expressed to enhance production of the hydrophobic compound (e.g., a polyisoprenoid) in the cells.

The vector of the present disclosure may be prepared by inserting a nucleotide sequence of a gene coding for the fusion protein, preferably a nucleotide sequence of a promoter and a nucleotide sequence of a gene coding for the fusion protein, into a vector commonly known as a transformation vector by conventional techniques.

Examples of vectors usable to prepare the vector of the present disclosure include pBI vectors, binary vectors such as pGA482, pGAH, and pBIG, intermediate plasmids such as pLGV23Neo, pNCAT, and pMON200, and pH35GS containing GATEWAY cassette.

Examples of promoters usable to prepare the vector of the present disclosure include promoters generally used in genetic recombination-related fields, such as CaMV35 and NOS promoters.

As long as the vector of the present disclosure contains a nucleotide sequence of a gene coding for the fusion protein, it may contain other nucleotide sequences. In addition to these nucleotide sequences, the vector usually contains sequences originated from the vector as well as other sequences such as a restriction enzyme recognition sequence, a spacer sequence, a marker gene sequence, and a reporter gene sequence.

Moreover, the vector of the present disclosure may contain a nucleotide sequence of a gene coding for a coenzyme as necessary.

Examples of the marker gene include drug-resistant genes such as a kanamycin-resistant gene, a hygromycin-resistant gene, and a bleomycin-resistant gene. Moreover, the reporter gene may be introduced to determine the expression site in a plant. Examples of the reporter gene include a luciferase gene, a β-glucuronidase (GUS) gene, a green fluorescent protein (GFP), and a red fluorescent protein (RFP).

### <Transgenic cell and method for producing substance using the transgenic cell>

A transgenic cell transformed to express the fusion protein can be obtained, for example, by introducing the vector of the present disclosure into a cell such as a plant cell. Thus, a transgenic cell into which a gene coding for the fusion protein has been introduced can be obtained. Then, in the transgenic cell in which the fusion protein is expressed, the functions including the certain enzymatic activity of the protein are newly exhibited in the cell with the introduced gene coding for the fusion protein, and thus the fusion protein binds to lipid droplets in the cell to synthesize the hydrophobic compound (e.g., a polyisoprenoid), thereby enhancing production of the hydrophobic compound (e.g., polyisoprenoid) in the cell. For example, when the second amino acid sequence used is an amino acid sequence derived from a cis-prenyltransferase family protein, the production of a polyisoprenoid (natural rubber) can be enhanced. Then, the synthesized hydrophobic compound is accumulated in the lipid droplets in the cell.

Moreover, a vector containing a nucleotide sequence of a gene coding for a coenzyme may be introduced, if necessary, together with the vector of the present disclosure into a cell such as a plant cell. Needless to say, a vector containing a nucleotide sequence of a gene coding for a coenzyme may be used as the vector of the present disclosure.

The host into which a gene coding for the fusion protein is to be introduced may be any organism containing lipid droplets. Examples include microorganisms such as algae and microalgae, plants, and animals. Among these, the host is preferably a microorganism or a plant, more preferably a microorganism, still more preferably a microalga.

In general, it is difficult to synthesize a hydrophobic compound using an organism. However, the synthesis of a hydrophobic compound can be facilitated when a gene coding for the fusion protein is introduced into the host described above.

The microorganisms may be either prokaryotes or eukaryotes. Examples include prokaryotes such as microorganisms of the genera *Escherichia, Bacillus, Synechocystis,* and *Synechococcus*; and eukaryotic microorganisms such as yeasts and filamentous fungi. Preferred among these are *Escherichia coli, Bacillus subtilis, Rhodosporidium toruloides,* and *Mortierella* sp., with *Escherichia coli* being more preferred.

Among the algae and microalgae, algae of the genera *Chlamydomonas, Chlorella, Phaeodactylum,* and *Nannochloropsis* are preferred because there are established genetic recombination techniques. More preferred are algae of the genus *Nannochloropsis.* Specific examples of the algae of the genus *Nannochloropsis* include *Nannochloropsis oculata, Nannochloropsis gaditana, Nannochloropsis salina, Nannochloropsis oceanica, Nannochloropsis atomus, Nannochloropsis maculata, Nannochloropsis granulata,* and *Nannochloropsis* sp. *Nannochloropsis oculata* or *Nannochloropsis gaditana* is preferred among these, with *Nannochloropsis oculata* being more preferred.

Among the plants, *Arabidopsis thaliana, Brassica napus, Brassica rapa, Cocos nucifera, Elaeis guineensis, Cuphea, Glycine max, Zea mays, Oryza sativa, Helianthus annuus, Cinnamomum camphora,* and *Jatropha curcas* are preferred, with *Arabidopsis thaliana* being more preferred.

A method for preparing the transgenic cell is briefly described below. The transgenic cell may be prepared by conventional techniques.

The vector of the present disclosure may be introduced into a plant (including plant cells, such as calluses, cultured cells, spheroplasts, and protoplasts) by any method that can introduce DNA into plant cells. Examples include methods using agrobacterium (JP S59-140885 A, JP S60-70080 A, WO94/00977), electroporation methods (JP S60-251887 A), and methods using particle guns (gene guns) (JP 2606856 B, JP 2517813 B).

In addition, the transgenic cell may be prepared by introducing the vector of the present disclosure into, for example, an organism (e.g., a microorganism, yeast, animal cell, or insect cell) or a part thereof, an organ, a tissue, a cultured cell, a spheroplast, or a protoplast, e.g., by any of the above-described DNA introduction methods.

The transgenic cell can be produced by the above or other methods. Here, the form of the transgenic cell is not limited as long as it includes a transformed cell. The transgenic cell may be a single cell or may be a tissue or transformant of combined cells. The transgenic cell conceptually includes not only transgenic cells produced by the above methods, but also all of their progeny or clones and even progeny organisms obtained by passaging these cells.

For example, once obtaining transgenic plant cells into which the vector of the present disclosure has been introduced, progeny or clones can be produced from the transgenic plant cells by sexual or asexual reproduction, tissue culture, cell culture, cell fusion, or other techniques. Moreover, the transgenic plant cells, or their progeny or clones may be used to obtain reproductive materials (e.g., seeds, fruits, cuttings, stem tubers, root tubers, shoots, adventitious buds, adventitious embryos, calluses, protoplasts), which may then be used to produce the transgenic plant on a large scale.

Techniques to regenerate plants (transgenic plants) from transgenic plant cells are known. For example, Doi et al. disclose techniques for eucalyptus (Japanese patent application No. H11-127025), Fujimura et al. disclose techniques for rice (Fujimura et al., (1995), Plant Tissue Culture Lett., vol. 2: p. 74-), Shillito et al. disclose techniques for corn (Shillito et al., (1989), Bio/Technology, vol. 7: p. 581-), Visser et al. disclose techniques for potato (Visser et al., (1989), Theor. Appl. Genet., vol. 78: p. 589-), and Akama et al. disclose techniques for *Arabidopsis thaliana* (Akama et al., (1992), Plant Cell Rep., vol. 12: p. 7-). A person skilled in the art can regenerate plants from transgenic plant cells according to these documents.

Whether a target protein gene is expressed in a regenerated plant may be determined by well-known methods. For example, Western blot analysis may be used to assess the expression of a target protein.

Seeds can be obtained from the transgenic plant, for example, as follows. The transgenic plant may be rooted in an appropriate medium and transplanted to water-containing soil in a pot. It may be grown under proper cultivation conditions to finally produce seeds, which are then collected. Moreover, plants can be grown from seeds, for example, as follows: the seeds obtained from the transgenic plant as described above may be sown in water-containing soil and grown under proper cultivation conditions into plants.

In the present disclosure, by introducing the vector of the present disclosure into cells such as plant cells, the gene coding for the fusion protein in the vector can be expressed to enhance production of the hydrophobic compound (e.g., a polyisoprenoid) in the cells. Specifically, the hydrophobic compound (e.g., a polyisoprenoid) may be produced by culturing, for example, transgenic cells produced as described above, calluses obtained from the transgenic plant cells, or cells redifferentiated from the calluses in an appropriate medium, or by growing, for example, transgenic plants redifferentiated from the transgenic plant cells, or plants grown from seeds collected from these transgenic plants under proper cultivation conditions.

Thus, another aspect of the present disclosure relates to a method of introducing the vector of the present disclosure into cells such as plant cells to enhance production of the hydrophobic compound (e.g., a polyisoprenoid) in the cells.

### (Method for producing rubber product)

The method for producing a rubber product of the present disclosure includes: producing a polyisoprenoid by the method for producing a substance (using a transgenic cell into which a gene coding for the fusion protein has been introduced); kneading the polyisoprenoid with an additive to obtain a kneaded mixture; forming a raw rubber product from the kneaded mixture; and vulcanizing the raw rubber product.

The rubber product is as described above in the present disclosure.

When the rubber product is a pneumatic tire or, in other words, when the method for producing a rubber product of the present disclosure is the method for producing a pneumatic tire of the present disclosure, the raw rubber product forming step corresponds to forming a green tire from the kneaded mixture, and the vulcanization step corresponds to vulcanizing the green tire. Thus, the method for producing a pneumatic tire of the present disclosure includes: producing a polyisoprenoid by the method for producing a substance (using a transgenic cell into which a gene coding for the fusion protein has been introduced); kneading the polyisoprenoid with an additive to obtain a kneaded mixture; forming a green tire from the kneaded mixture; and vulcanizing the green tire.

### <Kneading step>

In the kneading step, the polyisoprenoid produced by the method for producing a substance (using a transgenic cell into which a gene coding for the fusion protein has been introduced) is kneaded with an additive to obtain a kneaded mixture.

The polyisoprenoid produced using a transgenic cell into which a gene coding for the fusion protein has been introduced may be obtained by harvesting lipid droplets from the transgenic cell, and subjecting the harvested lipid droplets to a solidification step as described below.

The lipid droplets may be harvested from the transgenic cell by any method including usual methods. For example, the lipid droplets may be harvested by cutting a part of the transgenic cell, crushing the cut tissue, and extracting the crushed tissue with an organic solvent.

### <Solidification step>

The harvested lipid droplets are subjected to a solidification step. The solidification may be carried out by any method, such as by adding the lipid droplets to a solvent that does not dissolve the polyisoprenoid (natural rubber), such as ethanol, methanol, or acetone, or adding an acid to the lipid droplets. The rubber (natural rubber) can be recovered as solids from the lipid droplets by the solidification step. The recovered rubber (natural rubber) may be dried if necessary before use.

Any additive may be used including additives used in the production of rubber products. For example, in the case where the rubber product is a pneumatic tire, examples of the additive include rubber components other than the rubber obtained from the lipid droplets, reinforcing fillers such as carbon black, silica, calcium carbonate, alumina, clay, and talc, silane coupling agents, zinc oxide, stearic acid, processing aids, various antioxidants, softeners such as oils, waxes, vulcanizing agents such as sulfur, and vulcanization accelerators.

In the kneading step, kneading may be carried out using an open roll mill, a Banbury mixer, an internal mixer, or other rubber kneading machines.

### <Raw rubber product forming step (green tire forming step in the case of tire)>

The raw rubber product forming step is as described above in the present disclosure.

### <Vulcanization step>

The vulcanization step is as described above in the present disclosure.

### EXAMPLES

The present disclosure is specifically described with reference to examples, but the present disclosure is not limited to these examples.

### [Extraction of total RNA from Hevea latex]

Total RNA was extracted from the latex of *Hevea brasiliensis* by the hot phenol method. To 6 mL of the latex were added 6 mL of 100 mM sodium acetate buffer and 1 mL of a 10% SDS solution, and then 12 mL of water-saturated phenol pre-heated at 65°C. The mixture was incubated for five minutes at 65°C, agitated in a vortex mixer, and centrifuged at 7,000 rpm for 10 minutes at room temperature. After the centrifugation, the supernatant was transferred to a new tube, 12 mL of a phenol:chloroform (1:1) solution was added, and the mixture was agitated by shaking for two minutes. After the agitation, the resulting mixture was centrifuged again at 7,000 rpm for 10 minutes at room temperature. Then, the supernatant was transferred to a new tube, 12 mL of a chloroform:isoamyl alcohol (24:1) solution was added, and the mixture was agitated by shaking for two minutes. After the agitation, the resulting mixture was centrifuged again at 7,000 rpm for 10 minutes at room temperature. Then, the supernatant was transferred to a new tube, 1.2 mL of a 3M sodium acetate solution and 13 mL of isopropanol were added, and the mixture was agitated in a vortex mixer. The resulting mixture was incubated for 30 minutes at -20°C to precipitate total RNA. The incubated mixture was centrifuged at 15,000 rpm for 10 minutes at 4°C, and the supernatant was removed to collect a precipitate of total RNA. The collected total RNA was washed twice with 70% ethanol and then dissolved in RNase-free water.

### [Synthesis of cDNA from total RNA]

cDNA was synthesized from the collected total RNA. The cDNA synthesis was carried out using a PrimeScript II 1st strand cDNA synthesis kit (Takara) in accordance with the manual.

### [Acquisition of CPT, NgBR, and SRPP genes from cDNA]

The prepared 1st strand cDNA was used as a template to obtain CPT, NgBR, and SRPP genes. PCR was performed using a KOD-plus-Neo (Toyobo) in accordance with the manual. The PCR reaction involved 35 cycles with each cycle consisting of 10 seconds at 98°C, 30 seconds at 58°C, and 1 minute at 68°C.

The CPT gene was obtained using the following primers.
Primer 1: 5'-tttggatccgatggaattatacaacggtgagagg-3'
Primer 2: 5'-tttgcggccgcttattttaagtattccttatgtttctcc-3'

The NgBR gene was obtained using the following primers.
Primer 3: 5'-tttctcgagatggatttgaaacctggagctg-3'
Primer 4: 5'-tttctcgagtcatgtaccataattttgctgcac-3'

Moreover, a gene corresponding to the amino acids 1 to 81 of NgBR was obtained using the previously obtained NgBR gene as a template with the following primers.
Primer 33: 5'-aaaactcgagatggatttgaaacctggagc-3'
Primer 34: 5'-ttactagttcaagcttcttccactatctaccaca-3'

The SRPP gene was obtained using the following primers.
Primer 5: 5'-tttctcgagatggctgaagaggtggag-3'
Primer 6: 5'-tttggatccttatgatgcctcatctcc-3'

A CPT gene (HRT1), a NgBR gene (HRBP gene), and a SRPP gene were produced as described above. The genes were sequenced to identify the full-length nucleotide sequence and amino acid sequence. The nucleotide sequence of HRT1 is given by SEQ ID NO:1. The amino acid sequence of HRT1 is given by SEQ ID NO:2. Moreover, the nucleotide sequence of HRBP is given by SEQ ID NO:7. The amino acid sequence of HRBP is given by SEQ ID NO:6. Moreover, the nucleotide sequence of SRPP is given by SEQ ID NO:12. The amino acid sequence of SRPP is given by SEQ ID NO:11.

Moreover, a gene having a nucleotide sequence corresponding to the amino acid sequence at positions 1 to 81 (HRBP (1-81)) of the amino acid sequence (HRBP) represented by SEQ ID NO:6 was obtained.

### [Synthesis of Persea americana cDNA]

An avocado (Hass avocado) from Mexico, purchased from Shotei, was used. The avocado was preliminarily preserved at -80°C. The avocado was crushed in liquid nitrogen and the total RNA was extracted with TRIzol reagent (Invitrogen), followed by sugar precipitation. To 100 µL of the RNA solution was added 10 µL of 3M NaOAc, and the mixture was left on ice for one hour. Then, the mixture was centrifuged at 13,000 × g at 4°C for 15 minutes, and the supernatant was collected. To the supernatant was added 250 µL of 100% ethanol, and the mixture was left at - 30°C for one hour. The resulting mixture was again centrifuged at 13,000 × g at 4°C for 10 minutes, and the supernatant was collected. Thereto was added 70% ethanol, and the mixture was centrifuged at 13,000 × g at 4°C for five minutes. The supernatant was discarded, followed by drying. To the dried product was added 50 µL of DEPC. cDNA was synthesized from the obtained RNA using Fast Gene Scriptase II (NIPPON Genetics EUROPE).

### [Cloning of LDAP1 and LDAP2]

DNA fragments of LDAP1 and LDAP2 were amplified by PCR using the cDNA as a template and KOD-Plus-Neo (TOYOBO). The PCR was carried out using the following primer combinations.

The primers used for the LDAP1 gene were:
Primer 7: 5'-tcgaggatcccatggcagaagcagatgcaaaactgc-3' and
Primer 8: 5'-gcatactagttcaattgactacctcggatgtggtc-3'.

The primers used for the LDAP2 gene were:
Primer 9: 5'-tcgaggatcccatggcggaaaaagaaggaaggc-3' and
Primer 10: 5'-gcatactagttcattcagctgcaactgcaacg-3'.

The PCR-amplified product was separated by 0.8% agarose gel electrophoresis, and the gel was cut out, followed by purification using Fast Gene^{™} Gel/PCR Extraction Kit (Nippon GENETICS). The purified DNA fragment was inserted into a pGEM-T EASY vector by TA cloning. Next, *Escherichia coli* DH5α was transformed and then applied to LB agar medium and cultured overnight at 37°C, followed by blue-white screening. Multiple white colonies were selected to isolate a single colony into which a target sequence had been introduced. The colony was cultured on LB liquid medium containing ampicillin (Amp) (50 µg/mL Amp) at 37°C for 16 hours. The cultured cells were collected, and the plasmid was recovered using Fast Gene^{™} Plasmid Mini Kit (GENETICS). Finally, the nucleotide sequence was confirmed by DNA sequencing.

LDAP genes (LDAP1 and LDAP2) were produced as described above. The genes were sequenced to identify the full-length nucleotide sequence and amino acid sequence. The nucleotide sequence of LDAP1 is given by SEQ ID NO:8. The amino acid sequence of LDAP1 is given by SEQ ID NO:4. The nucleotide sequence of LDAP2 is given by SEQ ID NO:13. The amino acid sequence of LDAP2 is given by SEQ ID NO:10.

### [Acquisition of CPT gene from Arabidopsis thaliana]

A CPT gene (AtCPT5) was obtained from *Arabidopsis thaliana* in the same manner as described above. The gene was sequenced to identify the full-length nucleotide sequence and amino acid sequence. The nucleotide sequence of AtCPT5 is given by SEQ ID NO:9. The amino acid sequence of AtCPT5 is given by SEQ ID NO:3.

### [Preparation of construct]

### [[Preparation of LDAP1, LDAP2, and SRPP cell-free expression constructs]]

The LDAP1&2 genes and SRPP gene obtained above were each introduced into a cell-free expression plasmid (pEU-E01-His-TEV-MCS-N2 vector, CellFree Science, Matsuyama, Japan). The LDAP1&2 genes and the SRPP gene were introduced into the BamHI-SpeI site and the XhoI-BamHI site, respectively, in the multicloning site.

### [[Preparation of HRT1 and HRBP (1-81) cell-free expression constructs]]

The HRT1 and HRBP (1-81) genes obtained above were each introduced into a cell-free expression plasmid (pEU-E01-His-TEV-MCS-N2 vector). The HRT1 gene and the HRBP (1-81) gene were introduced into the BamHI-NotI site and the XhoI-SpeI site, respectively, in the multicloning site.

### [[Preparation of LDAP1-HRT1, LDAP2-HRT1, and SRPP-HRT1 cell-free expression constructs]]

To prepare a construct for a fusion protein in which a lipid droplet-binding protein was fused to the N-terminal side of HRT1, the LDAP1&2 and SRPP genes were again subjected to PCR using the primers shown below and introduced into the pEU-His-N2-HRT1 construct obtained by introducing HRT1 into the pEU-E01-His-TEV-MCS-N2 vector. The LDAP1 and SRPP genes and the LDAP2 gene were introduced into the EcoRV-BamHI site and the XhoI-BamHI site, respectively, in the multicloning site. Accordingly, a construct for a fusion protein in which a lipid droplet-binding protein was fused to the N-terminal side of HRT1 was successfully prepared.

The primers used in the amplification of the LDAP1 gene for fusion with HRT1 were:
Primer 11: 5'-agtcagatatctcatggcagaagcagatgcaaaac-3' and
Primer 12: 5'-tgactggatcctcattgactacctcggatgtggtc-3'.

The primers used in the amplification of the LDAP2 gene for fusion with HRT1 were:
Primer 13: 5'-agtcactcgagatggcggaaaaagaaggaagg-3' and
Primer 14: 5'-tgactggatcctcttcagctgcaactgcaacgtc-3'.

The primers used in the amplification of the SRPP gene for fusion with HRT1 were:
Primer 15: 5'-agtcagatatctcatggctgaagaggtggaggaag-3' and
Primer 16: 5'-tgactggatcctctgatgcctcatctccaaacacc-3'.

### [[Preparation of LDAP1-AtCPT5 and AtCPT5-LDAP1 cell-free expression constructs]]

Using the pEU-His-N2-LDAP1-HRT1 obtained by introducing LDAP1 into pEU-His-N2-HRT1, the AtCPT5 gene obtained as above was again subjected to PCR using the primers shown below, treated with BamHI and NotI, and introduced into the vector from which the HRT1 gene had been removed by treatment with the same enzymes, thereby introducing AtCPT5 to the C-terminal side of LDAP1 (LDAP1-AtCPT5) .

The primers used in the amplification of AtCPT5 gene for fusion with LDAP1 (in the following order: LDAP1-AtCPT5) were:
Primer 17: 5'-AGTCAGGATCCCATGTTGTCATTCTCTCTTCTC-3' and
Primer 18: 5'-TGACTGCGGCCGCGTCAAACCCGACAGCCAAATC-3'.

Moreover, PCR was again performed using the primers shown below so that the EcoRV and BamHI sites were at the ends of the AtCPT5 gene, and the BamHI and SpeI sites were at the ends of the LDAP1 gene, followed by introduction into a vector treated with the same enzymes, thereby introducing AtCPT5 to the N-terminal side of LDAP1 (AtCPT5-LDAP1) .

In the acquisition of AtCPT5 for fusion with LDAP1 (in the following order: AtCPT5-LDAP1), the primers used for AtCPT5 were:
Primer 19: 5'-agtcagatatctcatgttgtctattctctcttctcttttat-3' and
Primer 20: 5'-tgactggatcctcaacccgacagccaaatcg-3'.

The Primer 7 and Primer 8 were used for LDAP1.

### [[Preparation of LDAP1-linker-AtCPT5 and AtCPT5-linker-LDAP1 constructs]]

A pGFP-L4HPB plasmid used in Nobusawa et al.(2017)3 was provided by Prof. Hiroyuki Ohta, Biological system, Department of Life Science and Technology, School of Life Science and Technology, Tokyo Institute of Technology. A linker sequence in the plasmid was used as a template for PCR.

Here, a sequence present in a phmAG1-MNLinker vector (Medical & Biological Laboratories) was applied as the linker.

LDAP1 was linked to the linker sequence using the primers shown below by overlap extension PCR (LDAP1-linker, linker-LDAPl).

A LDAP1-linker fusion sequence was obtained using the following primers for LDAP1-linker fusion (overlap PCR).

The primers used on the LDAP1 side were:
Primer 21: 5'-tcgaggatcccatggcagaagcagatgcaaaactgc-3' and
Primer 22: 5'-cggaattaccggtattgactacctc-3'.

The primers used on the linker side were:
Primer 23: 5'-gaggtagtcaataccggtaattccg-3' and
Primer 24: 5'-tgactgcggccgcgccccttgggtcgatcctcc-3'.

A linker-LDAP1 fusion sequence was obtained using the following primers for linker-LDAP1 fusion (overlap PCR).

The primers used on the LDAP1 side were:
Primer 25: 5'-gacccaagggatggcagaag-3' and
Primer 26: 5'-gcatactagttcaattgactacctcggatgtggtc-3'.

The primers used on the linker side were:
Primer 27: 5'-agtcagcggccgcaccggtaattccgctgacg-3' and
Primer 28: 5'-cttctgccatcccttgggtc-3'.

For linking in the following order: LDAP1-linker-AtCPT5, the LDAP1-linker fusion sequence produced by overlap PCR was introduced between the BamHI and NotI sites of a pEU-E01-His-TEV-MCS-N2 vector, and further AtCPT5 was again subjected to PCR using the primers shown below to introduce AtCPT5 between the NotI and SpeI sites.

For linking in the following order: AtCPT5-linker-LDAP1, the linker-LDAP1 fusion sequence produced by overlap PCR was introduced between the NotI and SpeI sites of a pEU-E01-His-TEV-MCS-N2 vector, and AtCPT5 amplified using Primers 17 and 18 was introduced between the BamHI and NotI sites.

The primers used to amplify the AtCPT5 gene in the fusion of AtCPT5 with linker-LDAP1 were:
Primer 29: 5'-agtcagcggccgcatgttgtctattctctcttcctcttttatc-3' and
Primer 30: 5'-tgactactagttcaaacccgacagccaaatc-3'.

### [[Preparation of LDAP1-linker-HRT1 and HRT1-linker-LDAP1 constructs]]

These constructs were prepared as described above. Here, HRT1 was again subjected to PCR using the primers shown below before use.

The primers used to amplify the HRT1 gene in the fusion of HRT1 with LDAP1-linker were:
Primer 31: 5'-agtcagcggccgcatggaattatacaacggtgagagg-3' and
Primer 32: 5'-tgactactagtttattttaagtattccttatgtttctcc-3'.

When it was fused with linker-LDAP1, the linker-LDAP1 fusion sequence produced by overlap PCR was fused between the NotI and SpeI sites of the vector obtained by introducing the HRT1 gene into the BamHI-NotI site of a pEU-E01-His-TEV-MCS-N2 vector.

As to the linker sequence, the amino acid sequence represented by SEQ ID NO:5 was used and a gene having a nucleotide sequence corresponding to this amino acid sequence was used.

Here, it was confirmed that proteins having the combinations of the amino acid sequences shown in Table 6 and other tables were synthesizable using the constructs prepared above.

### [Preparation of lipid droplets (LD, oil droplets)]

Lipid droplets (LD) were extracted from the mesocarp of an avocado. First, 10 g of the mesocarp of an avocado *(Persea americana)* was crushed together with 20 mL (twice the amount of the avocado) of buffer A (shown in the table below) using a blender and then further disrupted in a mortar. The disrupted solution was filtered through Miracloth, and the filtrate was centrifuged at 15,000 × g at 4°C for 30 minutes. The fat pads floating on the upper layer were collected and resuspended. Thereto was added 5 mL of buffer B (shown in the table below), and the mixture was centrifuged at 15,000 × g at 4°C for 30 minutes. Again, the fat pads were collected and combined with buffer B, and the mixture was centrifuged at 15,000 × g at 4°C for 30 minutes. Next, to cause separation at the particle size of LD, the fat pads were collected, followed by centrifugation at 1,000 × g at 4°C for 10 minutes. Then, the lower layer solution was collected with a syringe. The solution was centrifuged at 15,000 × g at 4°C for 30 minutes to remove the lower layer. Then, 60 µL of TD buffer (shown in the table below) was added to obtain a LD solution.

**[Table 1]**

| | | Buffer A | | Buffer B | |
|---|---|---|---|---|---|
| | | volume | final | volume | final |
| 1 M Sucrose | | 18mL | 0.6 M | 8 mL | 0.4M |
| 0.1 M EDTA (pH 7.5) | | 0.3mL | 1 mM | 0.2 mL | 1 mM |
| 1 M KCl | | 0.3 mL | 10 mM | 0.2 mL | 10 mM |
| 1 M Tris-HCl (pH 7.5) | | 3 mL | 0.1 M | 2 mL | 0.1 M |
| SDW | | up to 30 mL | | up to 20 mL | |
| | | | TD Buffer volume | final | |
| | 1 M Tris-HCl | | 10 µL | 0.1 M | |
| | 1 M DTT | | 0.5 µL | 5 mM | |
| | SDW | | up to 100 µL | | |

### [Synthesis and extraction of mRNA in cell-free expression system]

mRNA was synthesized from the construct produced as described above using WEPRO7240H Expression Kit (ENDEXT Technology, CellFree Science). The reaction was performed using the following formulation at 37°C for three hours.

**[Table 2]**

| | volume | final |
|---|---|---|
| 5 × Transcription Buffer | 10 µL | 1× |
| 25 mM NTP mix | 6 µL | 3 mM |
| RNase Inhibitor (80,000 unit/mL) | 0.5 µL | |
| SR6 RNA Polymerase | 0.75 µL | 4 unit/50 µL |
| Plasmid | | 5 µg/50 µL |
| SDW | up to 50 µL | |

The reaction was followed by precipitation with ethanol. Finally, the precipitate was added to and dissolved in 25 µL of 1× DB buffer (shown in the table below) at room temperature. The collected mRNA was preserved at -80°C.

**[Table 3]**

| | final |
|---|---|
| HEPES-KOH (pH 7.8) | 120 mM |
| KOAc | 400 mM |
| Mg (Oac) ₂ | 10.8 mM |
| Spermidine | 1.6 mM |
| DTT | 10 mM |
| Amino acid mixture | 1.2 mM |
| ATP | 4.8 mM |
| GTP | 1 mM |
| Crphosphate | 64 mM |
| NaN₃ | 0.02% |

4× DB formulation

### [Preparation of purified LD by transcription reaction in cell-free expression system]

In order to express each protein on the LD purified as described above using the mRNA prepared as described above, a transcription reaction was performed using a wheat germ-derived cell-free protein expression kit (WEPRO7240H Expression Kit, ENDEXT Technology, CellFree Sciences).

First, 15 µL of 1× DB was added to 7.5 µL of the mRNA prepared as described above to prepare a mRNA premix. Then, a transcription reaction solution was prepared using the following formulation.

Here, the mRNA of a coenzyme was also added in the examples using the coenzyme in Table 6.

**[Table 4]**

| | volume |
|---|---|
| SDW | |
| 4×DB | 6.25 µL |
| LD | X µL |
| Creatine Kinase | 2 µL |
| RNase inhibitor | 1 µL |
| mRNA Premix | 17.5 µL |
| Wepro | 12.5 µL |
| total | 50 µL |

### Transcription reaction formulation

X: LD was prepared in an amount corresponding to 12.5 µg.

An amount of 650 µL of SDW was added to a plastic tube (PP container, 4.5 mL, 5-094-02, AS ONE). A dialysis cup (MWCO12000, COSMO BIO CO., LTD.) was placed in the tube so that air did not enter the dialysis membrane, and then allowed to stand for 10 minutes or longer. Then, the SDW was discarded from the tube, 650 µL of 1× DB buffer was added as an external fluid, and 50 µL of the prepared transcription reaction solution as an internal fluid was added into the dialysis cup. The dialysis cup was placed in the plastic tube so that air did not enter the dialysis membrane, and the cup was covered with a parafilm, followed by performing a reaction at 26°C for five hours. After the reaction, the external fluid was replaced with new 1× DB, and 5 µL of the mRNA premix was added to the internal fluid, followed by further reaction for 13 hours. After the reaction, the reaction solution was collected, 50 µL of which was transferred to a 1.5 mL tube, and 50 µL of 1× DB was added, followed by centrifugation at 15,000 × g at 4°C for 20 minutes. The LD layer and aqueous layer separated by centrifugation were transferred to a new tube, to which was added 50 µL of 1× DB, and the mixture was centrifuged at 15,000 × g at 4°C for 20 minutes. The aqueous layer was drawn out from the centrifuged solution using a syringe (TERUMO needle, NN-2719S, Terumo Corporation; 1 mL TERUMO tuberculin syringe, SS-01T, Terumo Corporation). The remaining LD layer was diluted with 100 µL of TD buffer to prepare a purified LD solution. Separately, to the tube from which the LD and aqueous layers were removed was added 100 µL of a buffer for resuspension to prepare a pellet solution.

### [Measurement of enzymatic activity for prenyl chain elongation]

The purified LD solution prepared as described above was shaken as the reaction composition shown below containing [4-¹⁴C]IPP (NEC773, Perkin Elmer) in a bath at 30°C for 18 hours. Here, to align the assay input of the purified LD solution, the protein concentration was measured by the Bradford method using Gene Spec, and an amount corresponding to 2 µg of proteins was input. In addition, in order to measure the background, ultrapure water was added instead of the purified LD solution to prepare a sample, which was then shaken as above.

**[Table 5]**

| | volume | final |
|---|---|---|
| Tris-HCl (pH 7.5) | 5 µL | 50 mM |
| DTT | 2 µL | 2 mM |
| MgCl₂ | 5 µL | 5 mM |
| KF | 2 µL | 20 mM |
| FPP | 3 µL | 15 µM |
| [4-¹⁴C]IPP (20 Ci/mol) | 10 µL | 50 µM |
| Purified LD solution | X µL | |
| Ultrapure water | up to 100 µL | |

Reaction composition for measurement of enzymatic activity for prenyl chain elongation

X: The purified LD was prepared in an amount corresponding to 2 µg.

After the reaction, 200 µL of saturated saline was added and stirred to terminate the reaction. To the reaction mixture was added 1 mL of saturated saline-saturated n-butanol, followed by agitation for one minute in a vortex mixer. The resulting mixture was centrifuged at 15,000 rpm for one minute at room temperature, and then the upper butanol layer was collected to extract a polyisoprenoid. An amount of 50 µL of the extract was added to 3 mL of clear-sol, and the radioactivity was measured using a liquid scintillation counter (LD6500, BECKMAN COULTER). The background value was subtracted from the measured value and, since 50 µL out of 1 mL was measured, the difference was multiplied by 20 times to calculate the count of the extract as a whole. A higher radioactivity (dpm) means a larger production of the polyisoprenoid (natural rubber), indicating a higher rubber synthesis activity.

Table 6 shows the results.

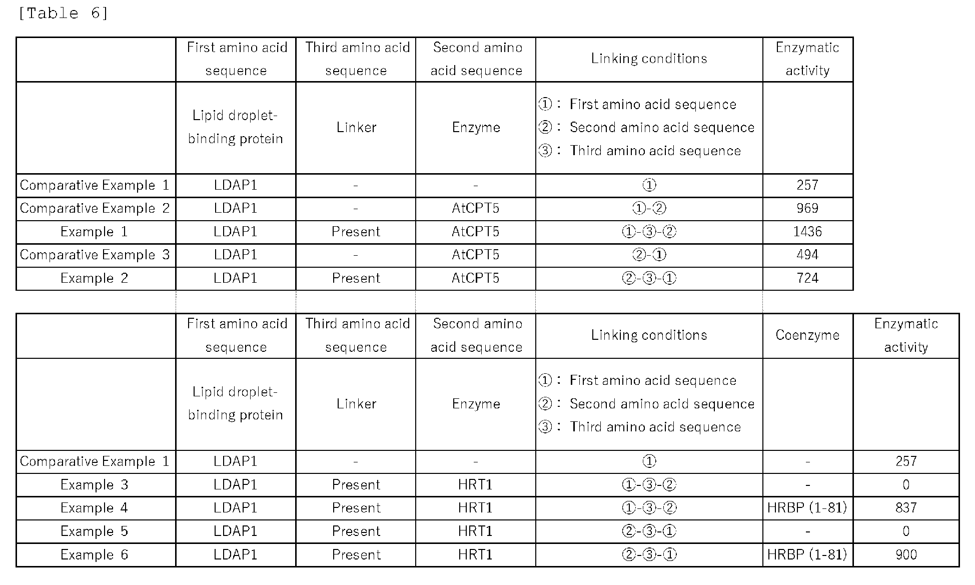

The upper section of Table 6 shows that the fusion proteins containing a linker sequence better maintained activity than the fusion proteins containing no linker sequence.

The lower section of Table 6 shows that HRT1 which alone exhibits no activity but exhibits activity by interacting with HRBP also did not lose its nature when it was fused with a lipid droplet-binding protein. In other words, it is demonstrated that the action of HRBP necessary for expressing the activity of HRT1 was not inhibited when it was in the form of a fusion protein, and that an enzyme requiring a cofactor was able to express activity when it was in the form of a fusion protein.

### [Electrophoresis test]

The reaction solution after protein expression or a lipid droplet fraction obtained by fractionating the reaction solution were subjected to SDS-PAGE electrophoresis to confirm expression of the protein and whether the protein was expressed on the purified LD. A mixture of 2 µL of a sample with 10 µL of a 2× SDS sample buffer and 8 µL of water was applied in 20 µL portions. The acrylamide concentration of the separation gel was 12% (w/v) or 15% (w/v), and Coomassie brilliant blue R-250 was used for staining.

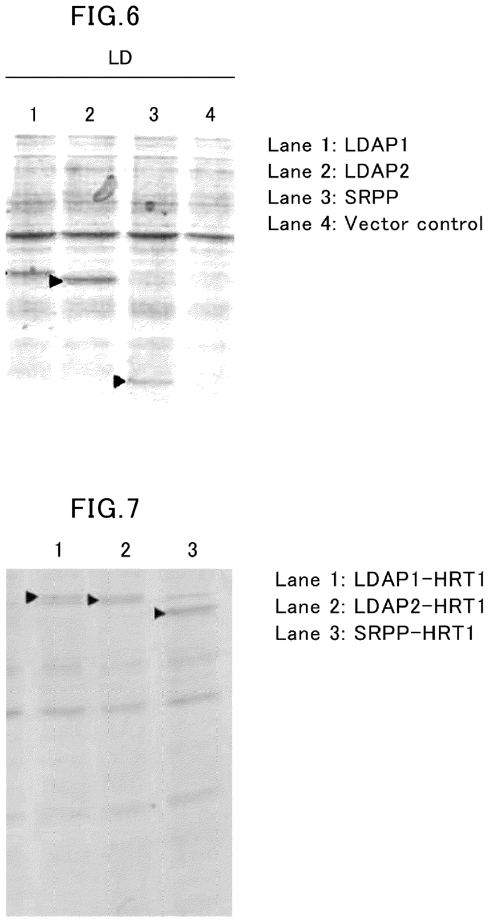

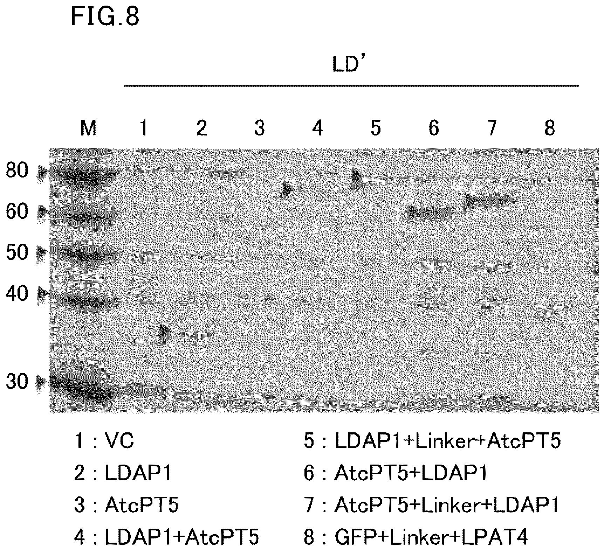

Figs. 6 to 8 show the results.

The results of the lipid droplet-binding ability test of LDAP1, LDAP2, and SRPP shown in Fig. 6 demonstrated that LDAP1, LDAP2, and SRPP bonded to lipid droplets.

The results of the lipid droplet-binding ability test of LDAP1, LDAP2, and SRPP fusion proteins shown in Fig. 7 demonstrated that all the LDAP1-HRT1, LDAP2-HRT1, and SRPP-HRT1 fusion proteins still exhibited lipid droplet-binding ability. This reveals that all the LDAP1/SRPP family proteins bonded to lipid droplets when they were in the form of a fusion protein with another protein.

The results of the lipid droplet-binding ability test of LDAP1-AtcPT5, LDAP1-linker-AtCPT5, AtCPT5-LDAP1, and AtCPT5-LDAP1 shown in Table 8 demonstrated that LDAP1 fused with a protein other than HRT1 (AtCPT5) maintained the lipid droplet-binding ability, and the ability was not lost by the presence of a linker or fusion order. This reveals that the (second amino acid) sequence of an enzyme binding to LDAP1 (lipid droplet-binding protein) is not limited, and the fusion order is not limited either. It is also revealed that AtCPT5 not fused with LDAP1 was not bound to lipid droplets.

The above results demonstrated that a fusion protein has an amino acid sequence (first amino acid sequence) capable of binding to lipid droplets, and an amino acid sequence (second amino acid sequence) having an enzymatic activity to synthesize a hydrophobic compound, and the first amino acid sequence and the second amino acid sequence are fused via a linker sequence (third amino acid sequence) while maintaining the enzymatic activity of the second amino acid sequence, and such a fusion protein can accumulate a hydrophobic compound (e.g., a polyisoprenoid) in lipid droplets by the enzymatic activity of the second amino acid sequence.

Moreover, binding of the fusion protein to the lipid droplets was also confirmed by the fact that the enzymatic activity was exhibited in the reaction test using the purified LD solution and also by the electrophoresis results.

### (SEQUENCE LISTING FREE TEXT)

SEQ ID NO:1: nucleotide sequence of gene coding for HRT1 from *Hevea brasiliensis*
SEQ ID NO:2: amino acid sequence of HRT1 from *Hevea brasiliensis*
SEQ ID NO:3: amino acid sequence of AtCPT5 from *Arabidopsis thaliana*
SEQ ID NO:4: amino acid sequence of LDAP1 from *Persea americana*
SEQ ID NO:5: amino acid sequence of linker sequence
SEQ ID NO:6: amino acid sequence of HRBP from *Hevea brasiliensis*
SEQ ID NO:7: nucleotide sequence of gene coding for HRBP from *Hevea brasiliensis*
SEQ ID NO:8: nucleotide sequence of gene coding for LDAP1 from *Persea americana*
SEQ ID NO:9: nucleotide sequence of gene coding for AtCPT5 from *Arabidopsis thaliana*
SEQ ID NO:10: amino acid sequence of LDAP2 from *Persea americana*
SEQ ID NO:11: amino acid sequence of SRPP from *Hevea brasiliensis*
SEQ ID NO:12: nucleotide sequence of gene coding for SRPP from *Hevea brasiliensis*
SEQ ID NO:13: nucleotide sequence of gene coding for LDAP2 from *Persea americana*
SEQ ID NO:14: Primer 1
SEQ ID NO:15: Primer 2
SEQ ID NO:16: Primer 3
SEQ ID NO:17: Primer 4
SEQ ID NO:18: Primer 5
SEQ ID NO:19: Primer 6
SEQ ID NO:20: Primer 7
SEQ ID NO:21: Primer 8
SEQ ID NO:22: Primer 9
SEQ ID NO:23: Primer 10
SEQ ID NO:24: Primer 11
SEQ ID NO:25: Primer 12
SEQ ID NO:26: Primer 13
SEQ ID NO:27: Primer 14
SEQ ID NO:28: Primer 15
SEQ ID NO:29: Primer 16
SEQ ID NO:30: Primer 17
SEQ ID NO:31: Primer 18
SEQ ID NO:32: Primer 19
SEQ ID NO:33: Primer 20
SEQ ID NO:34: Primer 21
SEQ ID NO:35: Primer 22
SEQ ID NO:36: Primer 23
SEQ ID NO:37: Primer 24
SEQ ID NO:38: Primer 25
SEQ ID NO:39: Primer 26
SEQ ID NO:40: Primer 27
SEQ ID NO:41: Primer 28
SEQ ID NO:42: Primer 29
SEQ ID NO:43: Primer 30
SEQ ID NO:44: Primer 31
SEQ ID NO:45: Primer 32
SEQ ID NO:46: Primer 33
SEQ ID NO:47: Primer 34

## Claims

1. A fusion protein, comprising:
an amino acid sequence (first amino acid sequence) capable of binding to lipid droplets; and
an amino acid sequence (second amino acid sequence) having an enzymatic activity to synthesize a hydrophobic compound,
the first amino acid sequence and the second amino acid sequence being fused via a linker sequence (third amino acid sequence) while maintaining the enzymatic activity of the second amino acid sequence.

2. The fusion protein according to claim 1,
wherein the first amino acid sequence is an amino acid sequence derived from a protein capable of binding to lipid droplets which is a class II protein.

3. The fusion protein according to claim 1 or 2,
wherein the second amino acid sequence is an amino acid sequence derived from an enzyme that has an enzymatic activity to synthesize a hydrophobic compound and that itself is not capable of binding to lipid droplets.

4. The fusion protein according to claim 1,
wherein the first amino acid sequence is an amino acid sequence derived from a lipid droplet-associated protein (LDAP)/small rubber particle protein (SRPP) family protein.

5. The fusion protein according to claim 1,
wherein the first amino acid sequence is an amino acid sequence derived from a LDAP/SRPP family protein of plant origin.

6. The fusion protein according to claim 1,
wherein the first amino acid sequence is an amino acid sequence derived from a LDAP/SRPP family protein derived from at least one selected from the group consisting of plants of the genera *Persea, Hevea,* and *Taraxacum.*

7. The fusion protein according to claim 1,
wherein the first amino acid sequence is an amino acid sequence derived from a LDAP/SRPP family protein derived from at least one plant selected from the group consisting of *Persea americana, Hevea brasiliensis,* and *Taraxacum kok-saghyz.*

8. The fusion protein according to any one of claims 1 to 7,
wherein the second amino acid sequence is an amino acid sequence derived from a prenyltransferase family protein.

9. The fusion protein according to any one of claims 1 to 7,
wherein the second amino acid sequence is an amino acid sequence derived from a cis-prenyltransferase family protein.

10. The fusion protein according to any one of claims 1 to 7,
wherein the second amino acid sequence is an amino acid sequence derived from a cis-prenyltransferase family protein derived from a plant of the genus *Hevea* or *Taraxacum.*

11. The fusion protein according to any one of claims 1 to 10,
wherein the third amino acid sequence consists of four or more amino acids.

12. The fusion protein according to any one of claims 1 to 10,
wherein the third amino acid sequence consists of 20 to 30 amino acids.

13. The fusion protein according to any one of claims 1 to 10,
wherein the third amino acid sequence is any one of the following amino acid sequences [1] to [3]:
[1] the amino acid sequence represented by SEQ ID NO:5;
[2] an amino acid sequence containing one or more amino acid substitutions, deletions, insertions, and/or additions relative to the amino acid sequence represented by SEQ ID NO:5; and
[3] an amino acid sequence with at least 80% sequence identity to the amino acid sequence represented by SEQ ID NO:5.

14. A method for producing a substance, the method comprising
binding the fusion protein according to any one of claims 1 to 13 to lipid droplets, and
accumulating a product in the lipid droplets by the enzymatic activity of the second amino acid sequence.

15. A vector into which a gene coding for the fusion protein according to any one of claims 1 to 13 has been introduced.

16. A transgenic cell into which a gene coding for the fusion protein according to any one of claims 1 to 13 has been introduced.

17. A method for producing a substance, the method comprising
using the transgenic cell according to claim 16 to accumulate a product in lipid droplets in the cell by the enzymatic activity of the second amino acid sequence.

18. A method for producing a pneumatic tire, the method comprising:
producing a polyisoprenoid by the method for producing a substance according to claim 14 or 17;
kneading the polyisoprenoid with an additive to obtain a kneaded mixture;
forming a green tire from the kneaded mixture; and
vulcanizing the green tire.

19. A method for producing a rubber product, the method comprising:
producing a polyisoprenoid by the method for producing a substance according to claim 14 or 17;
kneading the polyisoprenoid with an additive to obtain a kneaded mixture;
forming a raw rubber product from the kneaded mixture; and
vulcanizing the raw rubber product.


SEQUENCE LISTING

<110>  SUMITOMO RUBBER INDUSTRIES, LTD.
       TOHOKU UNIVERSITY
       KANAZAWA UNIVERSITY
      SAITAMA UNIVERSITY

<120>  FUSION PROTEIN, SUBSTANCE PRODUCTION METHOD, VECTOR, TRANSGENIC CELL,
PNEUMATIC TIRE PRODUCTION METHOD, AND RUBBER PRODUCT PRODUCTION METHOD

<130>  SR2822WO

<140>  PCT/JP2020/024525

<141>  2020-06-23

<150>  JP2019-131427

<151>  2019-07-16

<160>  47

<170>  PatentIn version 3.5

<210>  1
<211>  873
<212>  DNA
<213>  Hevea brasiliensis

<400>  1
atggaattat acaacggtga gaggccaagt gtgttcagac ttttagggaa gtatatgaga      60

aaagggttat atagcatcct aacccagggt cccatcccta ctcatattgc cttcatattg     120

gatggaaaca ggaggtttgc taagaagcat aaactgccag aaggaggtgg tcataaggct     180

ggatttttag ctcttctgaa cgtactaact tattgctatg agttaggagt gaaatatgcg     240

actatctatg cctttagcat cgataatttt cgaaggaaac ctcatgaggt tcagtacgta     300

atggatctaa tgctggagaa gattgaaggg atgatcatgg aagaaagtat catcaatgca     360

tatgatattt gcgtacgttt tgtgggtaac ctgaagcttt taagtgagcc agtcaagacc     420

gcagcagata agattatgag ggctactgcc aacaattcca aatgtgtgct tctcattgct     480

gtatgctata cttcaactga tgagatcgtg catgctgttg aagaatcctc tgaattgaac     540

tccaatgaag tttgtaacaa tcaagaattg gaggaggcaa atgcaactgg aagcggtact     600

gtgattcaaa ttgagaacat ggagtcgtat tctggaataa aacttgtaga ccttgagaaa     660

aacacctaca taaatcctta tcctgatgtt ctgattcgaa cttctgggga gacccgtctg     720

agcaactact tactttggca gactactaat tgcatactgt attctcctca tgcactgtgg     780

ccagagattg gtcttcgaca cgtggtgtgg gcagtaatta acttccaacg tcattattct     840

tacttggaga aacataagga atacttaaaa taa                                    873

<210>  2
<211>  230
<212>  PRT
<213>  Hevea brasiliensis

<400>  2

Gly Phe Leu Ala Leu Leu Asn Val Leu Thr Tyr Cys Tyr Glu Leu Gly
1               5                   10                  15

Val Lys Tyr Ala Thr Ile Tyr Ala Phe Ser Ile Asp Asn Phe Arg Arg
            20                  25                  30

Lys Pro His Glu Val Gln Tyr Val Met Asp Leu Met Leu Glu Lys Ile
        35                  40                  45

Glu Gly Met Ile Met Glu Glu Ser Ile Ile Asn Ala Tyr Asp Ile Cys
    50                  55                  60

Val Arg Phe Val Gly Asn Leu Lys Leu Leu Ser Glu Pro Val Lys Thr
65                  70                  75                  80

Ala Ala Asp Lys Ile Met Arg Ala Thr Ala Asn Asn Ser Lys Cys Val
                85                  90                  95

Leu Leu Ile Ala Val Cys Tyr Thr Ser Thr Asp Glu Ile Val His Ala
            100                 105                 110

Val Glu Glu Ser Ser Glu Leu Asn Ser Asn Glu Val Cys Asn Asn Gln
        115                 120                 125

Glu Leu Glu Glu Ala Asn Ala Thr Gly Ser Gly Thr Val Ile Gln Ile
    130                 135                 140

Glu Asn Met Glu Ser Tyr Ser Gly Ile Lys Leu Val Asp Leu Glu Lys
145                 150                 155                 160

Asn Thr Tyr Ile Asn Pro Tyr Pro Asp Val Leu Ile Arg Thr Ser Gly
                165                 170                 175

Glu Thr Arg Leu Ser Asn Tyr Leu Leu Trp Gln Thr Thr Asn Cys Ile
            180                 185                 190

Leu Tyr Ser Pro His Ala Leu Trp Pro Glu Ile Gly Leu Arg His Val
        195                 200                 205

Val Trp Ala Val Ile Asn Phe Gln Arg His Tyr Ser Tyr Leu Glu Lys
    210                 215                 220

His Lys Glu Tyr Leu Lys
225                 230

<210>  3
<211>  302
<212>  PRT
<213>  Arabidopsis thaliana

<400>  3

Met Leu Ser Ile Leu Ser Ser Leu Leu Ser Leu Leu Phe Leu Phe Ile
1               5                   10                  15

Ile Ser Cys Phe Phe Ile Thr Ser His Phe Trp Phe Pro Leu Ser Leu
            20                  25                  30

Pro Lys Ile Leu Gly Phe Ile Lys Ile Thr Ser Ser Arg Asp Asp Tyr
        35                  40                  45

Asp Asn Glu Gln Arg Asp Glu Gly Thr Tyr Val Val Gly Val Glu Glu
    50                  55                  60

Leu Gln Arg Glu Leu Met Pro Arg His Val Ala Val Ile Met Asp Gly
65                  70                  75                  80

Asn Arg Arg Trp Ala Lys Arg Ala Gly Leu Leu Thr Ser Gln Gly His
                85                  90                  95

Glu Ala Gly Ala Lys Arg Leu Ile Glu Phe Ser Glu Leu Cys Phe Lys
            100                 105                 110

Leu Gly Ile His Thr Val Ser Ala Phe Ala Phe Ser Thr Glu Asn Trp
        115                 120                 125

Gly Arg His Lys Ile Glu Val Lys Cys Leu Met Ser Leu Ile Gln His
    130                 135                 140

Tyr Leu Lys Ser Lys Ile Gln Tyr Phe Gln Arg Glu Glu Thr Arg Val
145                 150                 155                 160

Ser Val Ile Gly Asn Leu Thr Lys Ile Pro Glu Ser Leu Leu Arg Thr
                165                 170                 175

Val Gln Glu Ile Glu Glu Ala Thr Arg Ser Tyr Lys Lys Lys His Leu
            180                 185                 190

Ile Leu Ala Ile Asp Tyr Ser Gly Arg Leu Asp Ile Leu Arg Ala Cys
        195                 200                 205

Lys Ser Ile Val Lys Lys Ser Glu Lys Gly Leu Ile Arg Glu Glu Asp
    210                 215                 220

Val Asp Glu Ala Leu Ile Glu Arg Glu Leu Leu Thr Asn Cys Thr Glu
225                 230                 235                 240

Phe Pro Ser Pro Asp Leu Leu Ile Arg Thr Ser Gly Glu Gln Arg Ile
                245                 250                 255

Ser Asn Phe Phe Leu Trp Gln Leu Ala Tyr Thr Glu Leu Phe Phe Ser
            260                 265                 270

Pro Val Leu Trp Pro Asp Phe Asp Lys Asp Lys Leu Leu Glu Ala Leu
        275                 280                 285

Val Ser Tyr Gln Arg Arg Glu Arg Arg Phe Gly Cys Arg Val
    290                 295                 300

<210>  4
<211>  250
<212>  PRT
<213>  Persea americana

<400>  4

Met Ala Glu Ala Asp Ala Lys Leu Gln Asn Asp Thr Val Gln Glu Asp
1               5                   10                  15

Glu Val Thr Thr Leu Lys Tyr Leu Glu Phe Val Gln Ile Ala Ala Val
            20                  25                  30

His Ala Val Val Ile Val Ser Ser Ile Tyr Asp Phe Ala Lys Asp Asn
        35                  40                  45

Ser Gly Pro Leu Lys Pro Gly Val Gln Thr Val Glu Gly Thr Val Lys
    50                  55                  60

Thr Val Met Ser Pro Val Tyr Glu Lys Ile Gln Asp Ile Pro Leu Glu
65                  70                  75                  80

Leu Leu Lys Phe Ala Asp Arg Lys Val Asp Gly Phe Val Gln Glu Val
                85                  90                  95

Asp Arg His Val Pro Val Leu Val Lys Gln Ala Ser Ser Gln Ala Phe
            100                 105                 110

Ser Ala Ala His Arg Ala Pro Glu Val Ala Arg Ser Ile Lys Ser Glu
        115                 120                 125

Leu Asn Arg Val Gly Val Val Asp Thr Ala Thr Glu Met Thr Lys Ala
    130                 135                 140

Ala Tyr Ala Thr Leu Glu Pro Thr Ala Lys Asp Leu Tyr Ile Arg Tyr
145                 150                 155                 160

Glu Pro Val Ala Glu His Tyr Ala Val Ser Ala Trp Arg Ser Leu Asn
                165                 170                 175

Arg Leu Pro Leu Phe Ser Glu Val Ala His Ile Val Val Pro Thr Ala
            180                 185                 190

Ala Tyr Trp Thr Asp Lys Tyr Asn Lys Gly Val Arg Tyr Met Glu Val
        195                 200                 205

Lys Gly Phe Gly Val Ala Ser Tyr Met Pro Leu Val Pro Thr Glu Arg
    210                 215                 220

Ile Ala Lys Val Phe Gly Glu Arg Glu Glu Glu Ile Val Leu Glu Met
225                 230                 235                 240

Ser Pro Met Thr Thr Ser Glu Val Val Asn
                245                 250

<210>  5
<211>  24
<212>  PRT
<213>  Artificial Sequence

<400>  5

Thr Gly Asn Ser Ala Asp Gly Gly Gly Gly Ser Gly Gly Ser Gly Gly
1               5                   10                  15

Ser Gly Gly Gly Ser Thr Gln Gly
                20

<210>  6
<211>  257
<212>  PRT
<213>  Hevea brasiliensis

<400>  6

Met Asp Leu Lys Pro Gly Ala Gly Gly Gln Arg Val Asn Arg Leu Val
1               5                   10                  15

Asp Pro Ile Ser Tyr His Phe Leu Gln Phe Leu Trp Arg Thr Leu His
                20                  25                  30

Leu Leu Val Ser Leu Trp Tyr Leu Gln Val Ser Met Val Gln Met Ile
        35                  40                  45

Glu Gly Phe Leu Ile Ser Ser Gly Leu Val Lys Arg Tyr Gly Ala Leu
    50                  55                  60

Asp Ile Asp Lys Val Arg Tyr Leu Ala Ile Val Val Asp Ser Glu Glu
65                  70                  75                  80

Ala Tyr Gln Ile Ser Lys Val Ile Gln Leu Leu Lys Trp Val Glu Asp
                85                  90                  95

Met Gly Val Lys His Leu Cys Leu Tyr Asp Ser Lys Gly Val Leu Lys
                100                 105                 110

Thr Asn Lys Lys Thr Ile Met Glu Ser Leu Asn Asn Ala Met Pro Phe
        115                 120                 125

Glu Glu Ala Val Glu Lys Asp Val Leu Leu Asp Gln Lys Gln Met Thr
    130                 135                 140

Val Glu Phe Ala Ser Ser Ser Asp Gly Lys Glu Ala Ile Thr Arg Ala
145                 150                 155                 160

Ala Asn Val Leu Phe Met Lys Tyr Leu Lys Tyr Ala Lys Thr Gly Val
                165                 170                 175

Gly Lys Glu Glu Pro Cys Phe Thr Glu Asp Gln Met Asp Glu Ala Leu
                180                 185                 190

Lys Ala Ile Gly Tyr Lys Gly Pro Glu Pro Asp Leu Leu Leu Ile Tyr
        195                 200                 205

Gly Pro Val Arg Cys His Leu Gly Phe Ser Pro Trp Arg Leu Arg Tyr
    210                 215                 220

Thr Glu Met Val His Met Gly Pro Leu Arg Tyr Met Asn Leu Gly Ser
225                 230                 235                 240

Leu Lys Lys Ala Ile His Arg Phe Thr Thr Val Gln Gln Asn Tyr Gly
                245                 250                 255

Thr

<210>  7
<211>  774
<212>  DNA
<213>  Hevea brasiliensis

<400>  7
atggatttga aacctggagc tggagggcag agagttaatc gtttagtgga tccgattagt        60

tatcattttc ttcaatttct gtggcgtact ctacatcttc ttgtcagctt atggtacctt       120

caagttagta tggtccaaat gatcgaaggc tttctaatct ctagtggact tgtgaaacgc       180

tatggagccc tcgatattga caaggtccgg taccttgcca ttgtggtaga tagtgaagaa       240

gcttaccaaa tttctaaagt tattcagctt ttgaaatggg tggaagatat gggtgtgaaa       300

catttatgcc tctatgattc aaaaggagtt ctcaagacaa acaagaaaac catcatggag       360

agtttgaaca atgctatgcc atttgaggaa gcagttgaaa aagatgtttt actggaccag       420

aaacagatga ctgtggaatt tgcttccagc tccgatggaa aggaagcaat aaccagggca       480

gctaacgtac tctttatgaa gtatttgaag tatgctaaaa ctggtgtagg aaaggaagaa       540

ccatgcttta cagaagatca aatggatgag gcactaaaag ctataggtta caaaggcccg       600

gaacctgact tgctattaat ttatggacct gttagatgcc atctaggttt ctcaccgtgg       660

agacttcgat atactgagat ggtgcatatg ggacccttga ggtacatgaa cctcggttca       720

ctaaaaaagg ccattcacag gttcacaaca gtgcagcaaa attatggtac atga             774

<210>  8
<211>  753
<212>  DNA
<213>  Persea americana

<400>  8
atggcagaag cagatgcaaa actgcaaaac gacacggttc aagaagatga ggtgacgaca      60

ctgaagtatc tggagttcgt ccaaatcgca gccgtgcacg cggtggtgat cgtctcgagc     120

atctatgatt tcgccaagga caactcaggt ccgttgaaac ccggagtgca gacggtcgag     180

ggcaccgtta agaccgtcat gagtcccgtc tacgagaaaa tccaagacat cccccttgag     240

ctcctcaagt tcgccgatcg taaggtggat ggattcgtgc aagaggtgga caggcacgtg     300

ccagtgcttg tgaagcaggc atcgagccag gccttctcgg cggctcacag ggctccagag     360

gtggctcgat ctatcaagtc tgagttgaac cgtgtgggtg tggtggatac tgcaaccgag     420

atgaccaagg ctgcgtatgc cacgctcgag ccaacagcaa aggacctgta catcagatac     480

gagcccgtcg ccgagcacta cgcagtctcg gcgtggcggt cgctcaaccg gcttcccctc     540

ttttcggagg tggcccacat tgtggtaccc acagcggcct actggacgga caagtacaac     600

aagggggtaa ggtacatgga ggtcaagggg tttggcgtgg cctcgtatat gccactggtg     660

cctacggaga ggatcgccaa ggtgttcggt gagagggagg aggaaatcgt gctcgagatg     720

agcccaatga ccacatccga ggtagtcaat tga                                  753



<210>  9
<211>  909
<212>  DNA
<213>  Arabidopsis thaliana

<400>  9
atgttgtcta ttctctcttc tcttttatct ctcctttttc tgtttatcat ctcttgtttc      60

ttcatcacaa gccatttttg gttccctctt tccttgccaa aaatactcgg attcatcaaa     120

atcacatctt cgagagacga ttatgacaac gagcaacgtg acgagggaac ttatgtggta     180

ggagtggagg agctacaaag agagctgatg ccaagacatg tggcagtgat aatggacgga     240

aaccggagat gggccaaacg ggccggattg ctgacgtcac aaggccacga ggccggagct     300

aaacggctta tagagttctc cgagctttgc tttaaattgg ggattcatac agtttcagct     360

tttgccttct ccacagagaa ttggggaaga cacaagattg aggttaagtg cttgatgtct     420

ttgatccaac attacctcaa gtccaagatc caatatttcc aaagagagga aactcgagtt     480

tctgttatcg ggaacctaac gaagatccct gagtctctcc tccgaacagt ccaagagata     540

gaggaagcta cgagaagcta taagaagaag catctcatat tggcaataga ttacagcggg     600

agattagaca tcttgcgagc ttgcaagagt attgtgaaga aatcagaaaa agggttgatc     660

cgagaggaag atgtagacga ggcattgatc gaaagagagc ttctgacaaa ttgtactgag 720

ttcccaagtc ctgatctatt gattaggaca agtggagaac agaggattag taacttcttc 780

ttgtggcaac ttgcttatac agagctcttc ttctcgccgg tcctttggcc tgatttcgat 840

aaggataagc ttctagaggc cctggtttcg tatcagcgcc gggaaagacg atttggctgt 900

cgggtttga 909

<210> 10
<211> 251
<212> PRT
<213> Persea americana

<400> 10

Met Ala Glu Lys Glu Gly Arg Gln Gln Thr Glu Thr Val Gln Asp Arg
1               5                   10                  15

Glu Ile His Ser Leu Lys Tyr Leu Glu Phe Val Gln Leu Ala Ala Val
            20                  25                  30

His Ala Ile Val Phe Phe Trp Ser Ile Tyr Asn Phe Ala Lys Glu Asn
        35                  40                  45

Ser Gly Pro Leu Arg Pro Gly Val Gln Thr Val Glu Gly Thr Val Lys
    50                  55                  60

Thr Val Val Gly Pro Val Tyr Glu Lys Phe His Gly Val Pro Phe Lys
65                  70                  75                  80

Ile Leu Lys Phe Ala Asp Arg Lys Val Asp Glu Leu Val His Glu Val
                85                  90                  95

Asp Arg His Val Pro Ala Val Val Lys Gly Ala Ser Cys Gln Ala Phe
            100                 105                 110

Ser Ala Ala Arg Ser Val Ser Ser Glu Val Gln Arg Ala Gly Val Ile
        115                 120                 125

Asp Thr Ala Ala Glu Leu Ala Lys Val Ala Tyr Thr Arg Val Gln Pro
    130                 135                 140

Thr Val Lys Glu Leu Tyr Ile Arg Tyr Glu Pro Val Ala Glu Gly Tyr
145                 150                 155                 160

Ala Val Ser Ala Trp Arg Ser Leu Asn Arg Leu Pro Leu Phe Pro Glu

                165                 170                 175

Val Ala His Ile Val Val Pro Thr Ala Ala Tyr Trp Ser Glu Lys Tyr
            180                 185                 190

Asn Arg Ser Val Leu His Ile Ala Asp Lys Gly Tyr Met Val Ser Ala
        195                 200                 205

Tyr Met Pro Leu Val Pro Thr Glu Arg Ile Ala Lys Val Phe Gly Gly
    210                 215                 220

Glu Glu Asp Gly Ser Lys Leu Asp Ser Gly Arg Val Glu Pro Pro Pro
225                 230                 235                 240

Ser Ala Gly Ala Asp Val Ala Val Ala Ala Glu
                245                 250

<210> 11
<211> 204
<212> PRT
<213> Hevea brasiliensis

<400> 11

Met Ala Glu Glu Val Glu Glu Glu Arg Leu Lys Tyr Leu Asp Phe Val
1               5                   10                  15

Arg Ala Ala Gly Val Tyr Ala Val Asp Ser Phe Ser Thr Leu Tyr Leu
            20                  25                  30

Tyr Ala Lys Asp Ile Ser Gly Pro Leu Lys Pro Gly Val Asp Thr Ile
        35                  40                  45

Glu Asn Val Val Lys Thr Val Val Thr Pro Val Tyr Tyr Ile Pro Leu
    50                  55                  60

Glu Ala Val Lys Phe Val Asp Lys Thr Val Asp Val Ser Val Thr Ser
65                  70                  75                  80

Leu Asp Gly Val Val Pro Pro Val Ile Lys Gln Val Ser Ala Gln Thr
                85                  90                  95

Tyr Ser Val Ala Gln Asp Ala Pro Arg Ile Val Leu Asp Val Ala Ser
            100                 105                 110

Ser Val Phe Asn Thr Gly Val Gln Glu Gly Ala Lys Ala Leu Tyr Ala

115                 120                 125

Asn Leu Glu Pro Lys Ala Glu Gln Tyr Ala Val Ile Thr Trp Arg Ala
    130                 135                 140

Leu Asn Lys Leu Pro Leu Val Pro Gln Val Ala Asn Val Val Val Pro
145                 150                 155                 160

Thr Ala Val Tyr Phe Ser Glu Lys Tyr Asn Asp Val Val Arg Gly Thr
                165                 170                 175

Thr Glu Gln Gly Tyr Arg Val Ser Ser Tyr Leu Pro Leu Leu Pro Thr
            180                 185                 190

Glu Lys Ile Thr Lys Val Phe Gly Asp Glu Ala Ser
        195                 200

<210>  12
<211>  615
<212>  DNA
<213>  Hevea brasiliensis

<400>  12
atggctgaag aggtggagga agagaggcta aagtatttgg attttgtgcg agcggctgga      60

gtttatgctg tagattcttt ctcaactctc tacctttatg ccaaggacat atctggtcca     120

ttaaaacctg gtgtcgatac tattgagaat gtggtgaaga ccgtggttac tcctgtttat     180

tatattcccc ttgaggctgt caagtttgta gacaaaacgg tggatgtatc ggtcactagc     240

ctagatggcg ttgttccccc agttatcaag caggtgtctg cccaaactta ctcggtagct     300

caagatgctc caagaattgt tcttgatgtg gcttcttcag ttttcaacac tggtgtgcag     360

gaaggcgcaa aagctctgta cgctaatctt gaaccaaaag ctgagcaata tgcggtcatt     420

acctggcgtg ccctcaataa gctgccacta gttcctcaag tggcaaatgt agttgtgcca     480

accgctgttt atttctctga aaagtacaac gatgttgttc gtggcactac tgagcaggga     540

tatagagtgt cctcttattt gcctttgttg cccactgaga aaattactaa ggtgtttgga     600

gatgaggcat cataa                                                      615

<210>  13
<211>  756
<212>  DNA
<213>  Persea americana

<400>  13

atggcggaaa aagaaggaag gcagcaaact gagacggttc aagatcggga gattcatagt 60

ctgaaatatc tagagttcgt ccaactcgcg gccgtgcacg caatcgtgtt cttctggagc 120

atctacaact ttgcaaagga gaattcaggg ccgttgaggc ccggcgtgca gactgtagaa 180

ggcaccgtca agaccgtcgt cggtcccgtc tacgagaaat tccacggcgt tccgttcaag 240

atcctcaagt tcgccgaccg gaaggtggat gagctggtgc acgaggtgga caggcacgtg 300

cctgcggtgg tgaagggagc atcgtgccag gccttttctg cggctcggtc cgtgtcgtcc 360

gaggtccaac gtgcgggcgt aatcgatacg gcagcggagt tggccaaggt ggcgtacacc 420

agggtccagc cgacggtgaa agagctgtac atccggtacg agccggttgc cgagggctat 480

gccgtgtcgg cgtggcgctc gctcaatcgt ctccctctgt tccccgaggt ggcacacatt 540

gtggtaccca ccgcggccta ctggtctgag aagtacaaca ggagcgtact ccacattgcc 600

gacaaggggt atatggtgtc ggcatacatg cccctggtac ctacggagag gatcgcgaag 660

gtgttcggag gggaggaaga cgggtccaag ctggacagtg gtcgtgttga gccgccgcca 720

tcagcgggtg ccgacgttgc agttgcagct gaatga 756

<210> 14
<211> 34
<212> DNA
<213> Artificial Sequence

<220>
<223> PRIMER-1

<400> 14
tttggatccg atggaattat acaacggtga gagg 34

<210> 15
<211> 39
<212> DNA
<213> Artificial Sequence

<220>
<223> PRIMER-2

<400> 15
tttgcggccg cttattttaa gtattcctta tgtttctcc 39

<210> 16
<211> 31
<212> DNA
<213> Artificial Sequence

<220>
<223> PRIMER-3

<400> 16
tttctcgaga tggatttgaa acctggagct g 31

<210>  17
<211>  33
<212>  DNA
<213>  Artificial Sequence

<220>
<223>  PRIMER-4

<400>  17
tttctcgagt catgtaccat aattttgctg cac                                    33

<210>  18
<211>  27
<212>  DNA
<213>  Artificial Sequence

<220>
<223>  PRIMER-5

<400>  18
tttctcgaga tggctgaaga ggtggag                                           27

<210>  19
<211>  27
<212>  DNA
<213>  Artificial Sequence

<220>
<223>  PRIMER-6

<400>  19
tttggatcct tatgatgcct catctcc                                           27

<210>  20
<211>  36
<212>  DNA
<213>  Artificial Sequence

<220>
<223>  PRIMER-7

<400>  20
tcgaggatcc catggcagaa gcagatgcaa aactgc                                 36


<210>  21
<211>  35
<212>  DNA
<213>  Artificial Sequence

<220>
<223>  PRIMER-8

<400>  21
gcatactagt tcaattgact acctcggatg tggtc                                  35

<210>  22
<211>  33
<212>  DNA
<213>  Artificial Sequence

<220>
<223>  PRIMER-9

<400>  22
tcgaggatcc catggcggaa aaagaaggaa ggc                                    33

<210>  23
<211>  32
<212>  DNA
<213>  Artificial Sequence

<220>
<223>  PRIMER-10

<400>  23
gcatactagt tcattcagct gcaactgcaa cg                                     32

<210>  24
<211>  35
<212>  DNA
<213>  Artificial Sequence

<220>
<223>  PRIMER-11

<400>  24
agtcagatat ctcatggcag aagcagatgc aaaac                                  35


<210>  25
<211>  35
<212>  DNA
<213>  Artificial Sequence

<220>
<223>  PRIMER-12

<400>  25
tgactggatc ctcattgact acctcggatg tggtc                                  35

<210>  26
<211>  32
<212>  DNA
<213>  Artificial Sequence

<220>
<223>  PRIMER-13

<400>  26
agtcactcga gatggcggaa aaagaaggaa gg                                     32

<210>  27
<211>  34

<212>  DNA
<213>  Artificial Sequence

<220>
<223>  PRIMER-14

<400>  27
tgactggatc ctcttcagct gcaactgcaa cgtc                                34

<210>  28
<211>  35
<212>  DNA
<213>  Artificial Sequence

<220>
<223>  PRIMER-15

<400>  28
agtcagatat ctcatggctg aagaggtgga ggaag                               35

<210>  29
<211>  35
<212>  DNA
<213>  Artificial Sequence

<220>
<223>  PRIMER-16

<400>  29
tgactggatc ctctgatgcc tcatctccaa acacc                               35

<210>  30
<211>  33
<212>  DNA
<213>  Artificial Sequence

<220>
<223>  PRIMER-17

<400>  30
agtcaggatc ccatgttgtc attctctctt ctc                                 33

<210>  31
<211>  34
<212>  DNA
<213>  Artificial Sequence

<220>
<223>  PRIMER-18

<400>  31
tgactgcggc cgcgtcaaac ccgacagcca aatc                                34

<210>  32
<211>  41
<212>  DNA
<213>  Artificial Sequence

<220>
<223>  PRIMER-19

<400>  32
agtcagatat ctcatgttgt ctattctctc ttctctttta t                          41

<210>  33
<211>  31
<212>  DNA
<213>  Artificial Sequence

<220>
<223>  PRIMER-20

<400>  33
tgactggatc ctcaacccga cagccaaatc g                                     31

<210>  34
<211>  36
<212>  DNA
<213>  Artificial Sequence

<220>
<223>  PRIMER-21

<400>  34
tcgaggatcc catggcagaa gcagatgcaa aactgc                                36

<210>  35
<211>  25
<212>  DNA
<213>  Artificial Sequence

<220>
<223>  PRIMER-22

<400>  35
cggaattacc ggtattgact acctc                                            25

<210>  36
<211>  25
<212>  DNA
<213>  Artificial Sequence

<220>
<223>  PRIMER-23

<400>  36
gaggtagtca ataccggtaa ttccg                                            25

<210>  37
<211>  33
<212>  DNA
<213>  Artificial Sequence

<220>
<223>  PRIMER-24

<400>  37
tgactgcggc cgcgcccctt gggtcgatcc tcc                                    33

<210>  38
<211>  20
<212>  DNA
<213>  Artificial Sequence

<220>
<223>  PRIMER-25

<400>  38
gacccaaggg atggcagaag                                                   20

<210>  39
<211>  35
<212>  DNA
<213>  Artificial Sequence

<220>
<223>  PRIMER-26

<400>  39
gcatactagt tcaattgact acctcggatg tggtc                                  35


<210>  40
<211>  32
<212>  DNA
<213>  Artificial Sequence

<220>
<223>  PRIMER-27

<400>  40
agtcagcggc cgcaccggta attccgctga cg                                     32

<210>  41
<211>  20
<212>  DNA
<213>  Artificial Sequence

<220>
<223>  PRIMER-28

<400>  41
cttctgccat cccttgggtc                                                   20

<210>  42
<211>  43
<212>  DNA
<213>  Artificial Sequence

<220>
<223>  PRIMER-29

<400>  42
agtcagcggc cgcatgttgt ctattctctc ttcctctttt atc                         43

<210>  43
<211>  31
<212>  DNA
<213>  Artificial Sequence

<220>
<223>  PRIMER-30

<400>  43
tgactactag ttcaaacccg acagccaaat c                                    31

<210>  44
<211>  37
<212>  DNA
<213>  Artificial Sequence

<220>
<223>  PRIMER-31

<400>  44
agtcagcggc cgcatggaat tatacaacgg tgagagg                              37

<210>  45
<211>  39
<212>  DNA
<213>  Artificial Sequence

<220>
<223>  PRIMER-32

<400>  45
tgactactag tttattttaa gtattcctta tgtttctcc                            39

<210>  46
<211>  30
<212>  DNA
<213>  Artificial Sequence

<220>
<223>  PRIMER-33

<400>  46
aaaactcgag atggatttga aacctggagc                                      30


<210>  47
<211>  34
<212>  DNA
<213>  Artificial Sequence

<220>
<223>  PRIMER-34

<400>  47
ttactagttc aagcttcttc cactatctac caca                                 34